# EUROPÄISCHE PATENTSCHRIFT

(11) **EP 0 899 264 B1**
(45) Veröffentlichungstag und Bekanntmachung des Hinweises auf die Patenterteilung: **23.11.2005**
(21) Anmeldenummer: 98115651.6
(22) Anmeldetag: 20.08.1998
(51) Int. Cl.: C07D 243/08, A61K 31/55

(54) **7-Phenyl-1,4-diazepanderivate als Neurokininrezeptorantagonisten**
7-Phenyl-1,4-diazepane derivatives as neurokinine receptor antagonists
Dérivés de 7-phényl-1,4-diazépane comme antagonistes des récepteurs de neurokinine

(30) Priorität: 27.08.1997 DE 19737334
(43) Veröffentlichungstag der Anmeldung: 03.03.1999
(73) Patentinhaber: Solvay Pharmaceuticals GmbH, 30173 Hannover (DE)
(72) Erfinder: David, Samuel, 30559 Hannover (DE); Antel, Jochen, 31848 Bad Münder (DE); Brückner, Reinhard, 30559 Hannover (DE); Ziegler, Dieter, 30966 Hemmingen (DE); Eeckout, Christian, 29690 Lindwedel (DE); Bielenberg, Gerhard-Wilhelm, 31061 Alfeld (DE); Peck, Michael, 1440 Braine le Chateau (BE)
(74) Vertreter: Gosmann, Martin

(56) Entgegenhaltungen:
- EP-A- 0 655 442
- WO-A-96/34864
- DE-A- 2 328 870
- DE-A- 3 702 755
- PATENT ABSTRACTS OF JAPAN vol. 095, no. 009, 31. Oktober 1995 & JP 07 145060 A (TEIJIN LTD.), 6. Juni 1995
- PATENT ABSTRACTS OF JAPAN vol. 018, no. 482, 8. September 1994 & JP 06 157442 A (FUJI PHOTO FILM CO., LTD.), 3. Juni 1994

## Beschreibung

Die vorliegende Erfindung betrifft neue 7-Phenyl-1-benzoyl-1,4-diazepan-Derivate, welche in 4-Stellung durch eine einen N-Phenylalkyl-aminoalkyl-Rest oder einen N-Phenylalkyl-aminoalkylamino-Rest tragende Carbonylgruppe substituiert sind und deren Salze sowie diese Verbindungen enthaltende pharmazeutische Zubereitungen und Zwischenverbindungen und Verfahren zur Herstellung dieser Verbindungen.

Aus der europäischen Patentanmeldung, Veröffentlichungs-Nr. 0 655 442 sind 1,4-disubstituierte Piperazinderivate mit Tachykinin- und Neurokinin-Rezeptor-antagonistischen Wirkungen bekannt.

Neurokinine sind Neuropeptide, welche ebenso wie ihre zugehörigen Rezeptoren im menschlichen Körper weit verbreitet sind und im gastrointestinalen Trakt, im cardiovasculären Bereich und im ZNS-Bereich vorgefunden werden. Es handelt sich bei ihnen um Neurotransmitter, welche ein weitgefächertes Wirkungsspektrum besitzen und welche u. a. eine Rolle spielen bei Schmerzgeschehen, bei Entzündungsvorgängen, bei Gefäßerweiterungen und bei Kontraktionen der glatten Muskulatur, insbesondere im gastrointestinalen Bereich. Neurokinin-Rezeptor-Antagonisten sind pharmakologisch wirksame Substanzen, welche ein Bindungsvermögen an Neurokininrezeptoren besitzen und somit Neurokinin-induzierte Vorgänge hemmen können.

Aufgabe der vorliegenden Erfindung ist es, neue Neurokinin-Rezeptor-antagonistisch wirksame Verbindungen mit einem zur Behandlung von funktionellen und entzündlichen Störungen im gastrointestinalen Trakt günstigen Wirkungsprofil und guter Verträglichkeit zu entwickeln.

Es wurde nun gefunden, daß die erfindungsgemäßen, in 4-Stellung durch eine einen N-Phenylalkyl-aminoalkyl-Rest oder einen N-Phenylalkyl-aminoalkylamino-Rest tragende Carbonylgruppe substituierten 7-Phenyl-1-benzoyl-1,4-diazepan-Derivate Neurokinin-Rezeptor-antagonistische Wirkungen zeigen und zur Behandlung und Prophylaxe von durch an Neurokinin- bzw. Tachykinin-Rezeptoren bindende Stoffe verursachten pathologischen Zuständen geeignet sind und sich durch ein pharmakologisches Wirkungsprofil mit einer ausgeprägten Wirkkomponente gegenüber visceraler Schmerzüberempfindlichkeit und funktionellen Störungen im gastrointestinalen Bereich, insbesondere im Bereich der unteren Darmwege, auszeichnen.

Die Erfindung betrifft daher Verbindungen der allgemeinen Formel I 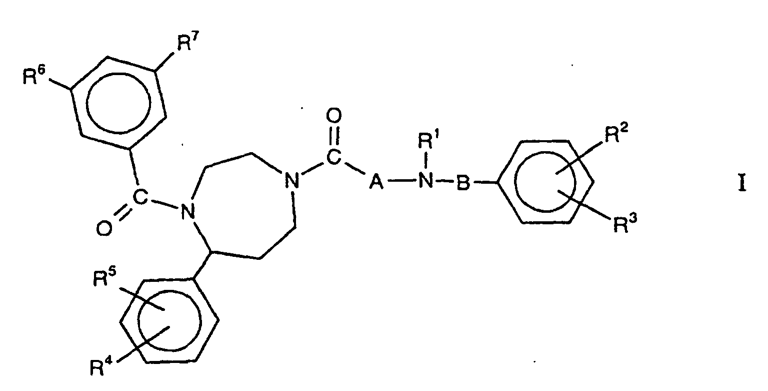 worin
- R¹: Wasserstoff oder niederes Alkyl bedeutet,
- R²: Wasserstoff, niederes Alkyl, niederes Alkoxy, Halogen oder Trifluormethyl bedeutet und
- R³: Wasserstoff, niederes Alkyl, niederes Alkoxy, Halogen oder Trifluormethyl bedeutet, oder ,
- R² und R³: gemeinsam an benachbarte Kohlenstoffatome des Phenylringes gebundenes Alkylendioxy mit 1 bis 2 Kohlenstoffatomen bedeuten,
- R⁴: Wasserstoff, niederes Alkyl, niederes Alkoxy, Halogen oder Trifluormethyl bedeutet und
- R⁵: Wasserstoff, niederes Alkyl, niederes Alkoxy, Halogen oder Trifluormethyl bedeutet, oder
- R⁴ und R⁵: gemeinsam an benachbarte Kohlenstoffatome des Phenylringes gebundenes Alkylendioxy mit 1 bis 2 Kohlenstoffatomen bedeuten,
- R⁶: niederes Alkyl, Halogen oder Trifluormethyl bedeutet,
- R⁷: niederes Alkyl, Halogen oder Trifluormethyl bedeutet,
- A: eine -(CH₂)ₙ-Gruppe, worin n für eine ganze Zahl von 1 bis 3 steht, oder eine -NH-(CH₂)ₘ-Gruppe, worin m für eine ganze Zahl von 2 bis 3 steht, bedeutet, und
- B: eine gegebenenfalls durch niederes Alkyl substituierte Alkylenkette mit 1 bis 3 Kohlenstoffatomen bedeutet,
sowie deren physiologisch verträgliche Säureadditionssalze.

Sofern in den Verbindungen der Formel I die Substituenten niederes Alkyl bedeuten oder enthalten, kann dieses verzweigt oder unverzweigt sein und vorzugsweise 1 bis 4 Kohlenstoffatome enthalten und ist bevorzugt Methyl.

Die Substituenten R² und R³ können jeweils unabhängig voneinander bevorzugt für Wasserstoff oder niederes Alkoxy, insbesondere Methoxy stehen. Sofern die Substituenten R² und/oder R³ für niederes Alkoxy stehen, kann der R² und R³ tragende Phenylring vorzugsweise einmal durch niederes Alkoxy substituiert sein und dieses kann insbesondere in 2-Position des Phenylringes angeordnet sein. Falls R² und/oder R³ für Halogen stehen, ist dieses insbesondere Chlor oder Fluor, vorzugsweise Fluor.

Sofern die Substituenten R⁴ und/oder R⁵ für Halogen stehen, ist Fluor bevorzugt. Vorzugsweise bedeuten R⁴ und R⁵ Wasserstoff.

Die Substituenten R⁶ und R⁷ stellen unabhängig voneinander jeweils bevorzugt Trifluormethyl dar. Falls R⁶ und/oder R⁷ niederes Alkyl bedeuten, ist dieses insbesondere Methyl. Falls R⁶ und/oder R⁷ für Halogen stehen, ist Chlor bevorzugt.

A kann vorzugsweise eine -(CH₂)ₙ-Gruppe sein. Vorzugsweise steht n für die Zahl 3.

Die Alkylenkette B ist bevorzugt unsubstituiert und stellt vorzugsweise die Methylengruppe dar.

Erfindungsgemäß können Verbindungen der allgemeinen Formel I erhalten werden, indem man
a) zur Herstellung von Verbindungen der allgemeinen Formel I Verbindungen der allgemeinen Formel IIa 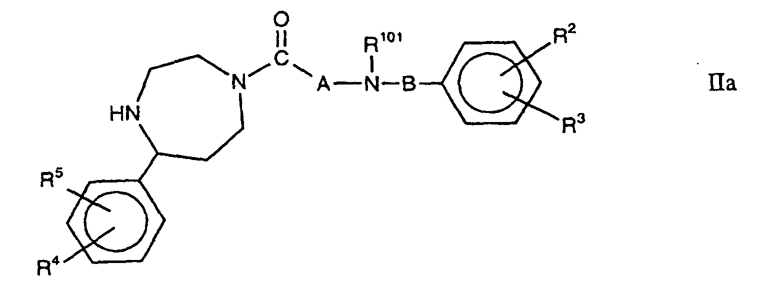 worin R², R³, R⁴, R⁵, A und B obige Bedeutungen besitzen und R¹⁰¹ für niederes Alkyl oder eine Aminoschutzgruppe steht, mit Verbindungen der allgemeinen Formel III 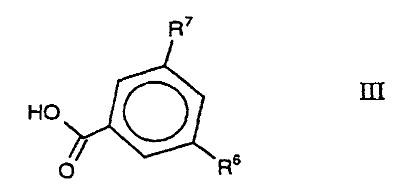 worin R⁶ und R⁷ obige Bedeutungen besitzen, umsetzt und eine allfällige Aminoschutzgruppe R¹⁰¹ nachfolgend wieder abspaltet,oder
b) zur Herstellung von Verbindungen der allgemeinen Formel Ia, 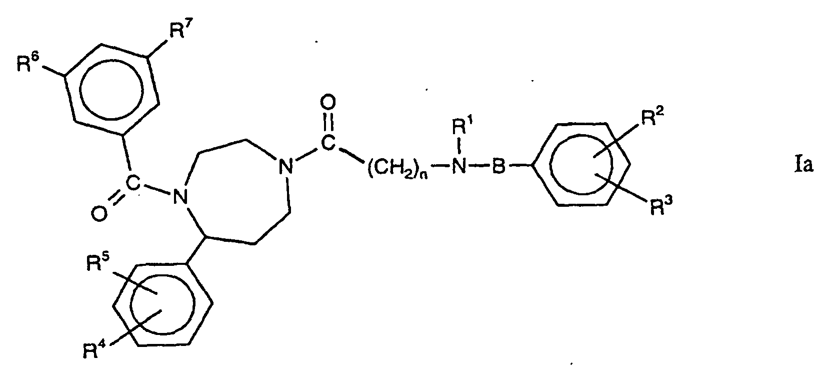 worin R¹, R², R³, R⁴, R⁵, R⁶, R⁷, B und n obige Bedeutungen besitzen, Verbindungen der allgemeinen Formel IV, 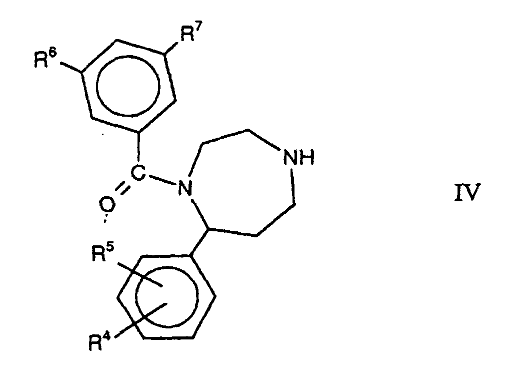 worin R⁴, R⁵, R⁶ und R⁷ obige Bedeutungen besitzen, mit Verbindungen der allgemeinen Formel V, 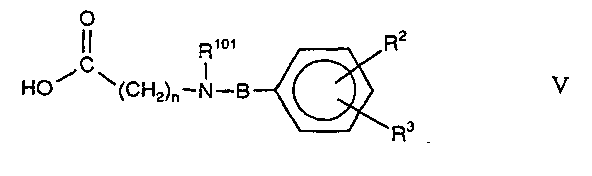 worin R¹⁰¹, R², R³, B und n obige Bedeutungen besitzen, umsetzt, und eine allfällige Aminoschutzgruppe R¹⁰¹ nachfolgend wieder abspaltet, oder
c) zur Herstellung von Verbindungen der allgemeinen Formel Ib, 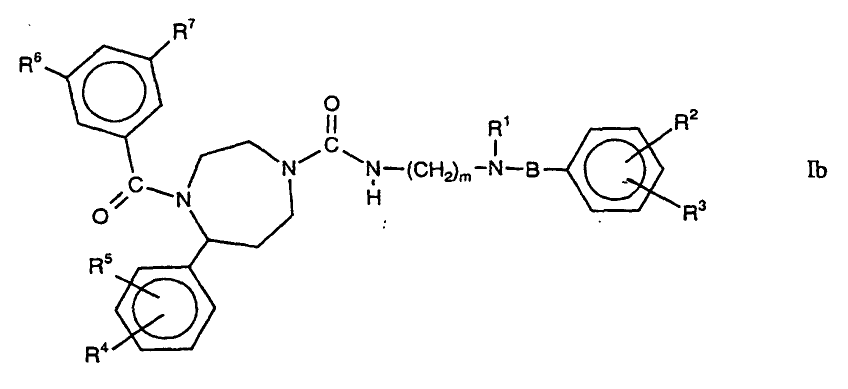 worin R¹, R², R³, R⁴, R⁵, R⁶, R⁷, B und m obige Bedeutungen besitzen, Verbindungen der Formel IV mit Verbindungen der allgemeinen Formel VI, 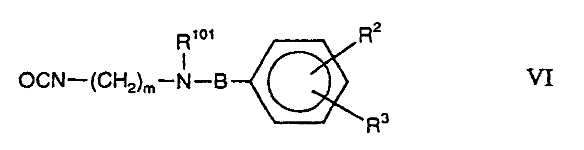 worin R¹⁰¹, R², R³, B und m obige Bedeutungen besitzen, umsetzt und eine allfällige Aminoschutzgruppe R¹⁰¹ nachfolgend wieder abspaltet, oder
d) zur Herstellung von Verbindungen der Formel I Verbindungen der allgemeinen Formel VIII, 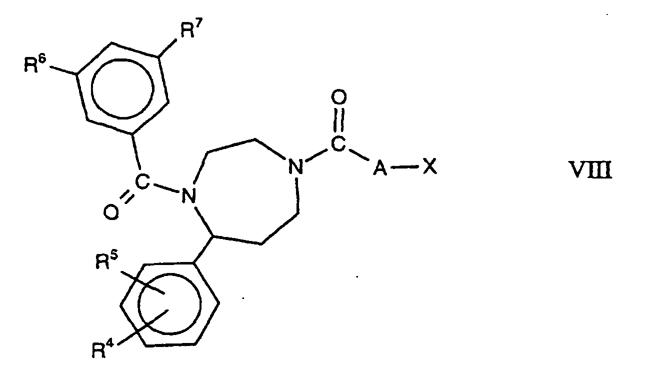 worin R⁴, R⁵, R⁶, R⁷ und A obige Bedeutungen besitzen und X für eine abspaltbare Fluchtgruppe steht, mit Verbindungen der allgemeinen Formel IXa, 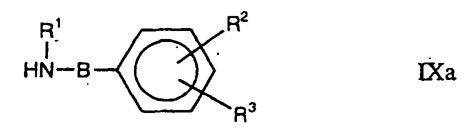 worin R¹, R², R³ und B obige Bedeutungen besitzen, umsetzt, oder
e) zur Herstellung von Verbindungen der Formel I Verbindungen der allgemeinen Formel X, 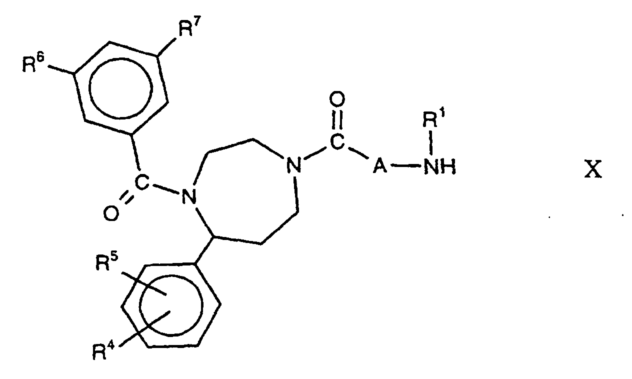 worin R¹, R⁴, R⁵, R⁶, R⁷ und A obige Bedeutungen besitzen, mit Verbindungen der allgemeinen Formel XIa, 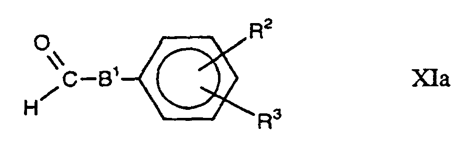 worin R² und R³ obige Bedeutungen besitzen und B¹ für eine Bindung oder eine gegebenenfalls durch niederes Alkyl substituierte Alkylenkette mit 1 bis 2 Kohlenstoffatomen steht, unter Bedingungen der reduktiven Alkylierung umsetzt oder mit Verbindungen der allgemeinen Formel XIb, 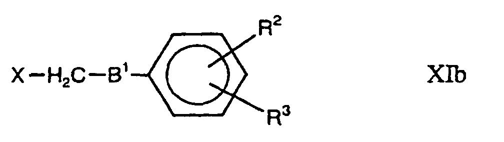 worin R², R³, B¹ und X obige Bedeutungen besitzen, alkyliert,
und gewünschtenfalls erhaltene Verbindungen der Formel I, worin R¹ Wasserstoff bedeutet, zu Verbindungen der Formel I, worin R¹ niederes Alkyl bedeutet, alkyliert und erhaltene Verbindungen der allgemeinen Formel I gewünschtenfalls in ihre Säureadditionssalze überführt oder Säureadditionssalze in freie Verbindungen der Formel I überführt.

Die Verbindungen der Formel I können auf an sich bekannte Weise gemäß Verfahrensvariante a) durch Umsetzung von Verbindungen der Formel IIa mit Verbindungen der Formel III unter zur Bildung von Amidgruppierungen durch Aminoacylierung üblichen Methoden und gegebenenfalls anschließender Abspaltung einer allfälligen Aminoschutzgruppe R¹⁰¹ hergestellt werden. Als Acylierungsmittel können die Säuren der Formel III oder deren reaktionsfähige Derivate eingesetzt werden. Als reaktionsfähige Derivate kommen insbesondere gemischte Säureanhydride und Säurehalogenide in Frage. So können beispielsweise Säurechloride oder Säurebromide der Säuren der Formel III oder gemischte Ester der Säuren der Formel III mit Chlorameisensäure oder mit organischen Sulfonsäuren, beispielsweise Niederalkansulfonsäuren wie z. B. Methansulfonsäure oder aromatischen Sulfonsäuren wie z. B. Benzolsulfonsäure oder durch niederes Alkyl oder Halogen substituierten Benzolsulfonsäuren, z. B. Toluolsulfonsäuren oder Brombenzolsulfonsäuren eingesetzt werden. Die Acylierung kann in einem unter den Reaktionsbedingungen inerten organischen Lösungsmittel, vorzugsweise bei Temperaturen zwischen -20 °C und Raumtemperatur erfolgen. Als Lösungsmittel eignen sich insbesondere aromatische Kohlenwasserstoffe wie Benzol oder Toluol, aliphatische Ether wie Diethylether, Tetrahydrofuran (im folgenden mit THF abgekürzt) oder Dioxan, teilhalogenierte niedere Kohlenwasserstoffe wie Dichlormethan oder Gemische dieser Lösungsmittel.

Die Acylierung kann zweckmäßig, insbesondere wenn als Acylierungsmittel ein Säurehalogenid der Säuren der Formel III verwendet wird, in Gegenwart eines säurebindenden Reagenzes durchgeführt werden. Als säurebindende Reagenzien eignen sich in dem Reaktionsgemisch lösliche nicht-nucleophile Basen, wie organische tertiäre Stickstoffbasen, beispielsweise stickstoffhaltige N-alkylierte Heterocyclen wie N-Niederalkyl-Morpholin oder N-Niederalkyl-Piperidin oder tertiäre Niederalkylamine und Pyridine, wie z. B. Triethylamin, Tripropylamin, Diisopropylethylamin, Pyridin, 4-Dimethylaminopyridin, 4-Diethylaminopyridin oder 4-Pyrrolidinopyridin. Im Überschuß eingesetzte organische Basen können gleichzeitig auch als Lösungsmittel verwendet werden.

Falls als Acylierungsmittel die Säuren der Formel III selbst eingesetzt werden, kann die Umsetzung der Amine der Formel IIa mit den Säuren der Formel III zweckmäßig auch in Gegenwart eines aus der Peptidchemie als zur Amidbildung geeignet bekannten Kopplungsreagenzes durchgeführt werden. Als Beispiel von Kopplungsreagenzien, welche die Amidbildung mit den freien Säuren dadurch fördern, daß sie mit der Säure in situ unter Bildung eines reaktionsfähigen Säurederivates reagieren, seien insbesondere genannt: Alkylcarbodiimide, z. B. Cycloalkylcarbodiimide wie Dicyclohexylcarbodiimid oder 1-Ethyl-3-[(dimethylamino)-propyl]-carbodiimid, Diisopropylcarbodiimid, Carbonyldiimidazol und N-Niederalkyl-2-halogen-pyridinium-Salze, insbesondere Halogenide oder Tosylate. Die Umsetzung in Gegenwart eines Kopplungsreagenzes kann zweckmäßig bei Temperaturen zwischen -30 °C und +50 °C in Lösungsmitteln wie halogenierten Kohlenwasserstoffen und/oder aromatischen Lösungsmitteln wie gegebenenfalls substituierten Benzolen, und gegebenenfalls in Gegenwart einer säurebindenden organischen Verbindung, beispielsweise einer vorstehend beschriebenen nicht-nucleophilen Stickstoffbase durchgeführt werden.

Die Herstellung von Verbindungen der Formel Ia gemäß Verfahrensvariante b) kann auf an sich bekannte Weise durch Umsetzung von Verbindungen der Formel IV mit Carbonsäuren der Formel V unter den oben für die Umsetzung von Verbindungen der Formel IIa mit Verbindungen der Formel III nach Verfahrensvariante a) angegebenen Bedingungen durchgeführt werden.

Verbindungen der Formel Ib können gemäß Verfahrensvariante c) hergestellt werden, indem man Verbindungen der Formel IV mit Isocyanaten der Formel VI in an sich bekannter Weise umsetzt. Die Verbindungen der Formel VI können beispielsweise aus den Aminen der allgemeinen Formel VII, 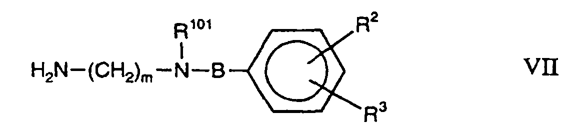 worin R¹⁰¹, R², R³, B und m obige Bedeutungen besitzen, auf an sich bekannte Weise durch Umsetzung mit geeigneten reaktiven Carbonylverbindungen erhalten werden. Als reaktive Carbonylverbindungen eignen sich beispielsweise Phosgen oder phosgenartig reagierende Substanzen wie Bis-(trichlormethyl)-carbonat (Triphosgen), Chlorameisensäure-trichlormethylester (Diphosgen) oder Carbonyldiimidazol. Zweckmäßig werden die Verbindungen der Formel Ib hergestellt, indem man aus Aminen der Formel VII zuerst Isocyanate der Formel VI herstellt und diese dann direkt in situ mit Verbindungen der Formel IV umsetzt. Die Reaktionsfolge kann als Eintopfreaktion in einem polaren aprotischen Lösungsmittel wie einem teilhalogenierten niederen Kohlenwasserstoff, beispielsweise Dichlormethan, bei Temperaturen zwischen -20 °C und Raumtemperatur, bevorzugt zwischen 0 °C und Raumtemperatur durchgeführt werden. Zweckmäßig kann dem Reaktionsgemisch ein säurebindendes Reagenz zugesetzt werden. Als säurebindende Reagenzien eignen sich die oben für die Umsetzung von Verbindungen der Formel IIa mit Verbindungen der Formel III angegebenen Reagenzien.

Isocyanate der Formel VI können auch aus Carbonsäuren der Formel V oder deren reaktiven Derivaten unter den Bedingungen eines Curtius-Abbaus hergestellt werden. So können beispielsweise nach allgemein üblichen Verfahren gewinnbare reaktive Derivate von Carbonsäuren der Formel V wie deren Ester, Anhydride oder Säurehalogenide, durch Umsetzung mit Alkalimetallaziden wie Natriumazid und nachfolgender Erwärmung auf an sich bekannte Weise in die entsprechenden Isocyanate überführt werden. Ebenso können die Säuren der Formel V durch Umsetzung mit Diphenylphosphorylazid oder einem ähnlich wirkenden Reagenz umgesetzt werden, um Isocyanate der Formel VI zu erhalten. Zweckmäßig kann auch diese Reaktionsfolge als Eintopf-Verfahren in einem unter den Reaktionsbedingungen inerten polaren, aprotischen Lösungsmittel, beispielsweise Dimethylformamid (im folgenden mit DMF abgekürzt), Dimethylsulfoxid (im folgenden mit DMSO abgekürzt) oder einem aliphatischen Ether wie THF oder Dioxan durchgeführt werden. Zweckmäßigerweise kann man dem Reaktionsgemisch zuerst bei Temperaturen zwischen 10 und 40 °C ein säurebindendes Reagenz zugeben und anschließend zur Vervollständigung des Umsatzes auf Temperaturen zwischen 80 und 120 °C, vorzugsweise auf 100 °C erhitzen. Als säurebindende Reagenzien können die für die Umsetzung von Verbindungen der Formel IIa mit Verbindungen der Formel III angegebenen Reagenzien verwendet werden.

Verbindungen der Formel I können gemäß Verfahrensvariante d) auch hergestellt werden, indem man Verbindungen der Formel VIII mit Verbindungen der Formel IXa unter für nucleophile Substitutionsreaktionen allgemein üblichen Bedingungen auf an sich bekannte Weise umsetzt. Als abspaltbare Fluchtgruppen X in Verbindungen der Formel VIII eignen sich Halogene, insbesondere Chlor, Brom und Iod oder ein organischer Sulfonsäurerest, beispielsweise der Rest einer Niederalkansulfonsäure wie Methansulfonsäure oder von aromatischen Sulfonsäuren wie Benzolsulfonsäure oder von durch niederes Alkyl oder Halogen substituierten Benzolsulfonsäuren wie Toluolsulfonsäuren. Die Reaktion kann in einem polaren aprotischen Lösungsmittel wie DMF, DMSO oder Acetonitril bei Temperaturen zwischen -20 °C und 100 °C, bevorzugt zwischen 60 °C und 90 °C und unter Verwendung eines säurebindenden Reagenzes durchgeführt werden. Als säurebindende Reagenzien eignen sich beispielsweise die oben für die Umsetzung von Verbindungen der Formel IIa mit Verbindungen der Formel III angegebenen säurebindenden Reagenzien.

Eine weitere Möglichkeit zur Herstellung der Verbindungen der Formel I ist die Alkylierung von Verbindungen der Formel X mit Verbindungen der Formel XIa oder XIb gemäß Verfahrensvariante e). Sofern Verbindungen der Formel XIa verwendet werden, kann die Umsetzung unter an sich zur reduktiven Alkylierung von Aminen üblichen Methoden durchgeführt werden. Dabei müssen die Reduktionsmittel und -bedingungen so gewählt werden, daß im Molekül vorhandene Amid-Carbonylgruppen nicht angegriffen werden. Beispielsweise kann die Reaktion unter den Bedingungen einer katalytischen Hydrierung durchgeführt werden. Die katalytische Hydrierung kann in einem unter den Reaktionsbedingungen inerten organischen Lösungsmittel wie einem niederen aliphatischen Ether, beispielsweise THF oder Diethylether, einem niederen Alkanol, beispielsweise Methanol oder Ethanol oder in Gemischen dieser Lösungsmittel und in Anwesenheit eines Hydrierungskatalysators durchgeführt werden. Als Hydrierungskatalysatoren eignen sich vorzugsweise Metallkatalysatoren wie Raney-Nickel. Zweckmäßig wird die Reaktion bei Raumtemperatur ausgeführt. Ein zur Hydrierung geeigneter Wasserstoffdruck kann zwischen Normaldruck und einem Wasserstoffdruck von 5 bar, bevorzugt zwischen 2 und 4 bar betragen.

Sofern Verbindungen der Formel XIb verwendet werden, kann die Umsetzung unter für nucleophilen Substitutionsreaktionen allgemein üblichen Bedingungen durchgeführt werden. So können beispielsweise die oben für Umsetzungen von Verbindungen der Formel VIII mit Verbindungen der Formel IXa nach Verfahrensvariante d) angegebenen Bedingungen gewählt werden.

Sofern bei der Herstellung von Verbindungen der Formel I oder von deren Zwischenprodukten freie Aminogruppen durch Aminoschutzgruppen geschützt werden, kommen im Rahmen der Erfindung an sich, beispielsweise aus der Peptidchemie, bekannte Aminoschutzgruppen in Frage, welche nach an sich bekannten Methoden eingeführt und wieder abgespalten werden können. Geeignete Schutzgruppen sind beispielsweise bekannt aus J.A.W. McOmie "Protective Groups in Organic Chemistry", Plenum Press 1973 oder T.W. Green und P.G.M. Wuts "Protective Groups in Organic Synthesis", Wiley and Sons 1991.

Bevorzugt können als Aminoschutzgruppen R¹⁰¹ im sauren und im alkalischen Milieu weitgehend stabile Gruppen verwendet werden, welche unter hydrogenolytischen Bedingungen wieder abgespalten werden können. Die Abspaltung der Schutzgruppe kann unter Bedingungen erfolgen, unter denen im Molekül gegebenenfalls anwesende gewünschte, gegebenenfalls im Phenylring substituierte Phenylniederalkylaminogruppen erhalten bleiben. Beispielsweise eignen sich als Aminoschutzgruppen R¹⁰¹ Phenylniederalkyloxycarbonylgruppen, vorzugsweise die Benzyloxycarbonylgruppe. Diese kann auf an sich bekannte Weise, z. B. durch katalytische Hydrierung, abgespalten werden, um Verbindungen der Formel I zu erhalten, worin R¹ Wasserstoff bedeutet. Die Reaktion kann in einem unter den Reaktionsbedingungen inerten organischen Lösungsmittel wie einem niederen aliphatischen Ether, beispielsweise THF oder Diethylether, niederen Alkanolen, beispielsweise Methanol oder Ethanol, oder organischen Säuren, beispielsweise niederen aliphatischen Carbonsäuren wie Essigsäure, oder in Gemischen dieser Lösungsmittel und in Anwesenheit eines Hydrierungskatalysators erfolgen. Als Hydrierungskatalysatoren eignen sich Edelmetallkatalysatoren wie Palladium auf Aktivkohle. Zweckmäßig wird die Reaktion bei Raumtemperatur ausgeführt. Ein zur Hydrierung geeigneter Wasserstoffdruck beträgt zwischen 3 und 7 bar, bevorzugt zwischen 4 und 6 bar.

Die Verbindungen der Formel I, worin R¹ Wasserstoff bedeutet, können gewünschtenfalls nach an sich zur Aminoalkylierung bekannten Methoden in Verbindungen der Formel I überführt werden, worin R¹ niederes Alkyl bedeutet. Hierfür können die Verbindungen der Formel I beispielsweise durch Umsetzung mit niederen aliphatischen Aldehyden wie Formaldehyd unter den oben für die Umsetzung von Verbindungen der Formel X mit Verbindungen der Formel XIa angegebenen Bedingungen reduktiv alkyliert werden. Eine andere Möglichkeit der Alkylierung ist die Umsetzung von Verbindungen der Formel I, worin R¹ Wasserstoff bedeutet, mit niederen aliphatischen Alkylhalogeniden wie Alkylbromiden oder Alkyliodiden, vorzugsweise Methyliodid, Alkylsulfaten oder Alkylsulfonsäureestern nach der oben für die Umsetzung von Verbindungen der Formel VIII mit Verbindungen der Formel IXa angegebenen Methode. Als säurebindende Reagenzien eignen sich die oben für die Umsetzung von Verbindungen der Formel IIa mit Verbindungen der Formel III beschriebenen säurebindenden Reagenzien.

Als physiologisch verträgliche Salze von Verbindungen der Formel I kommen deren Salze mit anorganischen Säuren, beispielsweise Schwefelsäure, Phosphorsäuren oder Halogenwasserstoffsäuren, vorzugsweise Chlorwasserstoffsäure, oder mit organischen Säuren, beispielsweise niederen aliphatischen Mono-, Di- oder Tricarbonsäuren wie Maleinsäure, Fumarsäure, Milchsäure, Weinsäure, Zitronensäure, oder mit Sulfonsäuren, beispielsweise Niederalkansulfonsäuren wie Methansulfonsäure oder gegebenenfalls im Benzolring durch Halogen oder niederes Alkyl substituierte Benzolsulfonsäuren wie p-Toluolsulfonsäure, in Frage.

Die Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in pharmakologisch verträgliche Säureadditionssalze überführt werden.

Die Verbindungen der Formel I enthalten ein chirales Kohlenstoffatom, nämlich das den durch R⁴ und R⁵ substituierten Phenylring tragende Kohlenstoffatom in 7-Stellung des 1,4-Diazepan-Grundgerüstes. Sofern B für eine durch niederes Alkyl ein- oder mehrfach substituierte Alkylenkette steht, kann wenigstens ein weiteres Chiralitätszentrum hinzutreten. Die Verbindungen der Formel I können somit in mehreren stereoisomeren Formen vorliegen. Die vorliegende Erfindung umfaßt sowohl die Gemische optischer Isomere wie auch die isomerenreinen Verbindungen der Formel I.

Falls bei der Synthese der Verbindungen der Formel I Gemische optischer Isomere der Ausgangsverbindungen der Formeln IIa, IV, VIII oder X eingesetzt werden, werden auch die Verbindungen der Formel I in Form von Gemischen optischer Isomere erhalten. Ausgehend von stereochemisch einheitlichen Ausgangsverbindungen können auch stereochemisch einheitliche Verbindungen der Formel I erhalten werden. Aus den Gemischen optischer Isomere können die stereochemisch einheitlichen Verbindungen der Formel I in an sich bekannter Weise erhalten werden, z. B. können Diastereomere der Formel I durch chromatographische Trennmethoden oder durch fraktionierte Kristallisation aufgetrennt werden und Enantiomere der Formel I können z. B. erhalten werden durch chromatographische Trennung an chiralen Trennmaterialien.

Die Verbindungen der Formel II, 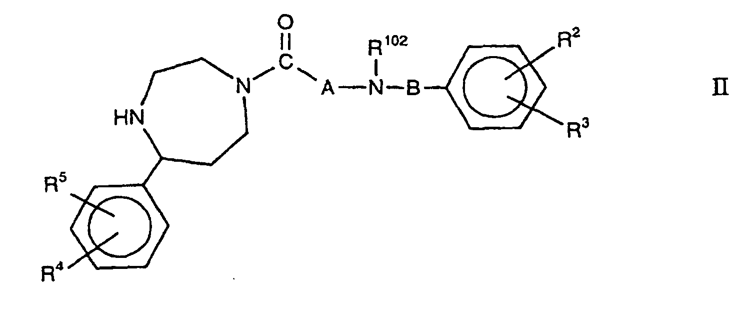 worin R², R³, R⁴, R⁵, A und B obige Bedeutungen besitzen und R¹⁰² Wasserstoff, niederes Alkyl oder eine Aminoschutzgruppe bedeutet, sind neue Verbindungen und stellen wertvolle Zwischenprodukte zur Herstellung von pharmazeutisch wirksamen Verbindungen, z. B. den Verbindungen der Formel I dar. Die Verbindungen der Formel II können auf an sich bekannte Weise erhalten werden.

So können Verbindungen der Formel IIa, worin A für eine -(CH₂)ₙ-Gruppe steht, worin n obige Bedeutung besitzt, durch Umsetzung von Carbonsäuren der Formel V oder deren reaktiven Derivaten mit Verbindungen der allgemeinen Formel XIIa, 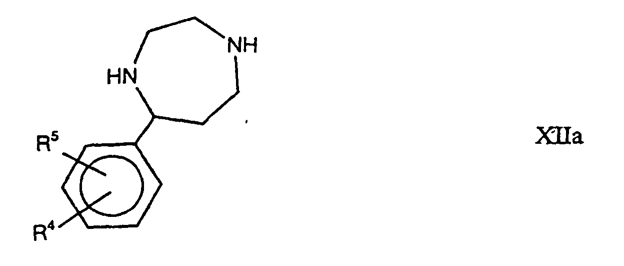 worin R⁴ und R⁵ obige Bedeutungen besitzen, beispielsweise nach dem oben für die Acylierung von Aminen der Formel IIa mit Carbonsäuren der Formel III angegebenen Verfahren erhalten werden.

Verbindungen der Formel IIa, worin A für eine -NH (CH₂)ₘ-Gruppe steht, worin m obige Bedeutung besitzt, können hergestellt werden, indem man Verbindungen der Formel VI mit Verbindungen der allgemeinen Formel XIIb, 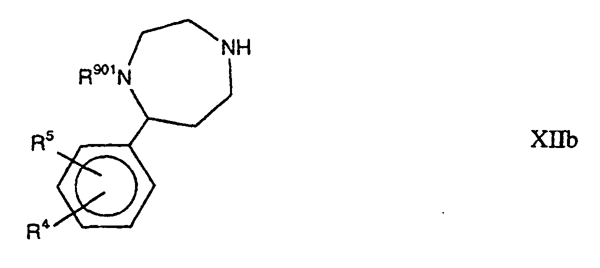 worin R⁴ und R⁵ obige Bedeutungen besitzen und R⁹⁰¹ für eine Aminoschutzgruppe steht, nach dem oben für die Umsetzung von Verbindungen der Formel IV mit Verbindungen der Formel VI angegebenen Verfahren umsetzt und die Schutzgruppe R⁹⁰¹ nachfolgend wieder abspaltet. Als Schutzgruppen R⁹⁰¹ eignen sich vorzugsweise im sauren Milieu, beispielsweise durch Zugabe von p-Toluolsulfonsäure, Trifluoressigsäure oder gasförmiger oder wäßrig gelöster Salzsäure selektiv abspaltbare Gruppen, welche gegen reduktive, insbesondere hydrogenolytische und alkalische Bedingungen weitgehend stabil sind. Hierzu zählen beispielsweise die Triphenylmethyl-(= Trityl)Gruppe sowie verzweigte Niederalkyloxycarbonylgruppen wie die tert.-Butyloxycarbonylgruppe. Vorzugsweise kann die tert.-Butyloxycarbonylgruppe (im folgenden als BOC-Gruppe abgekürzt)als Aminoschutzgruppe R⁹⁰¹ eingesetzt werden.

Die Zwischenprodukte der Formel IIa können auch durch Umsetzung von Verbindungen der allgemeinen Formel XIII, 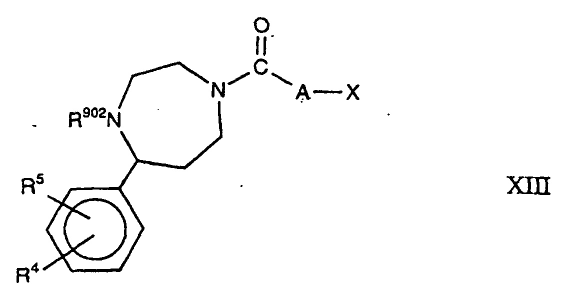 worin R⁴, R⁵, A und X obige Bedeutungen besitzen und R⁹⁰² für eine Aminoschutzgruppe steht, mit Verbindungen der allgemeinen Formel IXb, 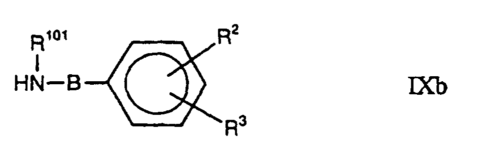 worin R¹⁰¹, R², R³ und B obige Bedeutungen besitzen, nach dem oben für die Umsetzung von Verbindungen der Formel VIII mit den Aminen der Formel IXa angegebenen Verfahren und nachfolgender Abspaltung der Aminoschutzgruppe R⁹⁰² hergestellt werden. Als Schutzgruppe R⁹⁰² eignen sich vorzugsweise durch katalytische Hydrierung wieder abspaltbare Aminoschutzgruppen. Als Hydrierungskatalysatoren hierfür sind beispielsweise Edelmetallkatalysatoren wie Palladium auf Aktivkohle oder Palladiumhydroxyd auf Aktivkohle geeignet. Vorzugsweise kann R⁹⁰² die Benzylgruppe sein. Gewünschtenfalls kann aus Verbindungen der Formel IIa, worin R¹⁰¹ für eine Aminoschutzgruppe steht, die Aminoschutzgruppe zur Freisetzung der -NH-Gruppe auf an sich bekannte Weise abgespalten werden, wodurch Verbindungen der Formel II erhalten werden können, worin R¹ Wasserstoff ist. Die Verbindungen der Formel IXb, worin R¹⁰¹ für eine Aminoschutzgruppe steht, stellen geschützte Derivate der Amine der Formel IXa dar und können aus Aminen der Formel IXa, worin R¹ für Wasserstoff steht, durch an sich bekannte Einführung einer Schutzgruppe R¹⁰¹ hergestellt werden. Die Amine der Formel IXa sind bekannt oder können auf an sich bekannte Weise aus bekannten Verbindungen hergestellt werden.

Die Verbindungen der Formel IV sind neu und stellen wertvolle Zwischenprodukte zur Herstellung von pharmazeutisch wirksamen Verbindungen, beispielsweise der Verbindungen der Formel 1 dar. Die Verbindungen der Formel IV können nach an sich bekannten Methoden hergestellt werden.

So können die Diazepan-Derivate der Formel IV beispielsweise durch an sich bekannte Reduktion der Diazepanon-Derivate der allgemeinen Formel XIV, 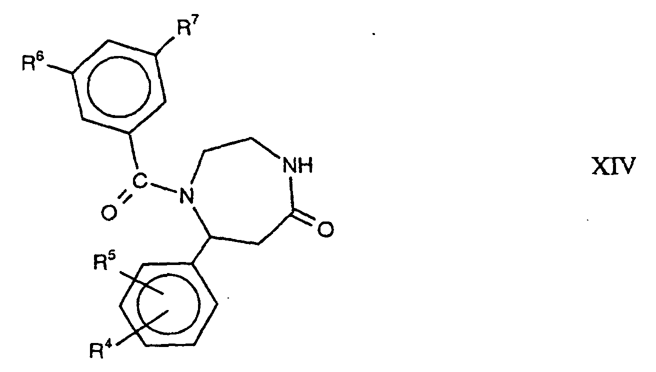 worin R⁴, R⁵, R⁶ und R⁷ obige Bedeutungen besitzen, erhalten werden. Die Reduktion der im Diazepan-Ringgerüst enthaltenen Carbonylgruppe kann selektiv durchgeführt werden, indem man die Verbindungen der Formel XIV zuerst mit einem Alkylierungsreagenz wie einem Triniederalkyloxoniumsalz, beispielsweise Triethyloxonium-Tetrafluoroborat, versetzt und das bei der Umsetzung entstehende Zwischenprodukt anschließend mit dem Reduktionsmittel umsetzt, wobei die eingeführte Alkylgruppe wieder abgespalten wird. Als Reduktionsmittel können Alkalimetallborhydride wie Natriumborhydrid verwendet werden. Die Umsetzung mit dem Alkylierungsmittel kann in einem aprotischen Lösungsmittel wie einem partiell halogenierten niederen Kohlenwasserstoff, beispielsweise Dichlormethan, einem Niederalkylcyanid, beispielsweise Acetonitril, oder einem Diniederalkylether wie Dioxan, THF oder Diethylether durchgeführt werden. Die Reaktionstemperatur kann zweckmäßigerweise zwischen -20 °C und ca. 60 °C, vorzugsweise bei Raumtemperatur liegen. Vorteilhaft ist es, das durch Reaktion mit dem Alkylierungsreagenz entstandene Zwischenprodukt zu isolieren, beispielsweise durch mindestens teilweises Abdampfen des ursprünglichen Lösungsmittels, und dieses anschließend in einem polaren protischen Lösungsmittel wie einem niederen Alkanol, beispielsweise Methanol oder Ethanol, erneut zu lösen. Eine zur Durchführung des Reduktionsschrittes geeignete Temperatur liegt zwischen -20 °C und 60 °C, bevorzugt kann bei Raumtemperatur gearbeitet werden.

Die Verbindungen der Formel VIII sind neu und stellen wertvolle Zwischenprodukte zur Herstellung von pharmazeutisch wirksamen Verbindungen, z. B. den Verbindungen der Formel I dar. Die Verbindungen der Formel VIII können nach an sich bekannten Methoden hergestellt werden.

So können beispielsweise Verbindungen der Formel VIII, worin A für eine -(CH₂)ₙ-Gruppe steht, worin n obige Bedeutung besitzt, hergestellt werden, indem man Verbindungen der Formel IV mit an sich bekannten ω-Halogencarbonsäuren der allgemeinen Formel XV, 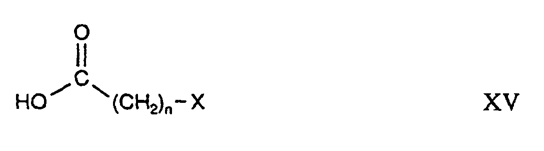 worin X und n obige Bedeutungen besitzen, oder deren reaktiven Derivaten, nach dem oben für die Umsetzung von Carbonsäuren der Formel III mit den Aminen der Formel IIa angegebenen Verfahren umsetzt.

Verbindungen der Formel VIII, worin A für eine -NH (CH₂)ₘ-Gruppe steht, worin m obige Bedeutung besitzt, können hergestellt werden, indem man Verbindungen der Formel IV mit Isocyanaten der allgemeinen Formel XVI,

OCN-(CH₂)ₘ-X XVI

worin X und m obige Bedeutungen besitzen, nach dem oben für die Umsetzung von Isocyanaten der Formel VI mit Aminen der Formel IV angegebenen Verfahren umsetzt. Die Isocyanate der Formel XVI sind bekannt oder können nach bekannten Methoden aus den entsprechenden Aminen der allgemeinen Formel XVII,

H₂N-(CH₂)ₘ-X XVII

worin X und m obige Bedeutungen besitzen, hergestellt werden. Beispielsweise können die Amine der Formel XVII nach der oben für die Umsetzung der Amine der Formel VII zu Isocyanaten der Formel VI beschriebenen Weise in die Isocyanate der Formel XVI überführt werden.

Die Verbindungen der Formel X sind neu und stellen wertvolle Zwischenprodukte zur Herstellung von pharmazeutisch wirksamen Verbindungen, z. B. den Verbindungen der Formel I dar. Die Verbindungen der Formel X können nach an sich bekannten Verfahren hergestellt werden.

So können beispielsweise Verbindungen der Formel X, worin A für eine -(CH₂)ₙ-Gruppe steht, worin n obige Bedeutung besitzt, hergestellt werden, indem man die Amine der Formel IV mit Carbonsäuren der allgemeinen Formel XVIIIa, 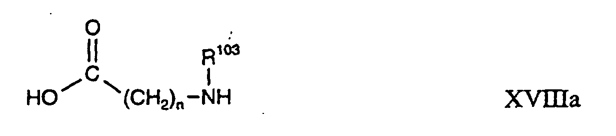 worin n obige Bedeutung besitzt und R¹⁰³ die für R⁹⁰¹ angegebene Bedeutung besitzt, nach dem oben für Umsetzungen der Amine der Formel IIa mit Carbonsäuren der Formel III angegebenen Verfahren umsetzt, und die Schutzgruppe R¹⁰³ anschließend auf an sich bekannte Weise abspaltet. Die Säuren der Formel XVIIIa stellen amino-geschütze ω-Aminocarbonsäuren dar, welche in ungeschützter Form bekannt sind und welche nach an sich bekannten Methoden hergestellt werden können.

Verbindungen der Formel X, worin A für eine -(CH₂)ₙ-Gruppe steht, worin n obige Bedeutung besitzt, können auch hergestellt werden, indem man Verbindungen der Formel III mit Verbindungen der allgemeinen Formel XIX, 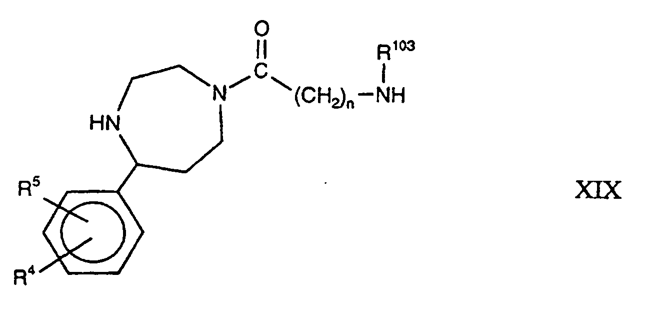 worin R¹⁰³, R², R³, und n obige Bedeutungen besitzen, umsetzt und die Schutzgruppe R¹⁰³ anschließend auf an sich bekannte Weise wieder abspaltet. Die Umsetzung kann nach an sich zur Amidbildung bekannten Methoden ausgeführt werden, beispielsweise kann die Reaktion nach dem oben für die Umsetzung von Verbindungen der Formel IIa mit Verbindungen der Formel III angegebenen Verfahren durchgeführt werden.

Verbindungen der Formel X, worin A für eine -NH-(CH₂)ₘ-Gruppe steht, worin m obige Bedeutung besitzt, können hergestellt werden, indem man die Amine der Formel IV mit Isocyanaten der allgemeinen Formel XX, 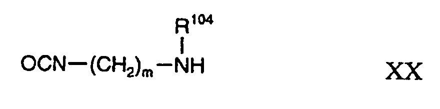 worin m obige Bedeutung besitzt und R¹⁰⁴ die für R⁹⁰¹ oder R⁹⁰² angegebene Bedeutung besitzen kann, nach dem oben für die Umsetzung der Amine der Formel IV mit den Isocyanaten der Formel VI beschriebenen Verfahren umsetzt und die Schutzgruppe R¹⁰⁴ anschließend auf an sich bekannte Weise wieder abspaltet. Die Isocyanate der Formel XX können nach dem oben für die Herstellung der Isocyanate der Formel VI aus den Aminen der Formel VII angegebenen Verfahren aus den Aminen der allgemeinen Formel XXI, 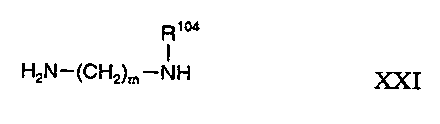 worin R¹⁰⁴ und m obige Bedeutungen besitzen, hergestellt werden. Die Verbindungen der Formel XXI stellen einfach aminogeschützte 1-ω-Diaminoalkane dar, welche in ungeschützter Form allgemein bekannt sind und welche nach an sich bekannten Methoden aus den ungeschützten Vorläuferverbindungen hergestellt werden können. Beispielsweise können die einfach amino-geschützten Amine der Formel XXI aus den entsprechenden ungeschützten Diaminoalkanen erhalten werden, indem man ein Moläquivalent des Diamins mit einem Moläquivalent des zur Einführung der Schutzgruppe benötigten Reagenzes umsetzt.

Die zur Herstellung von Verbindungen der Formel IIa verwendeten Carbonsäuren der Formel V können beispielsweise auf an sich zur reduktiven Alkylierung von Aminen bekannte Weise aus den ω-Aminocarbonsäuren der allgemeinen Formel XVIIIb, 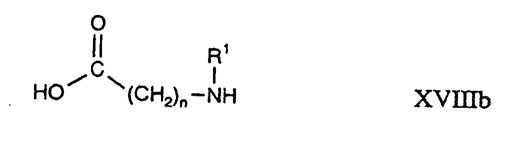 worin R¹ und n obige Bedeutungen besitzen, durch Umsetzung mit den Aldehyden der allgemeinen Formel XIa und, sofern R¹ für Wasserstoff steht, nachfolgender Einführung einer Aminoschutzgruppe R¹⁰¹, hergestellt werden. Beispielsweise kann die reduktive Alkylierung in wäßriger alkalischer Lösung, etwa in 1-normaler wäßriger Natronlauge durchgeführt werden. Die Zugabe eines Lösungsvermittlers wie eines wasserlöslichen organischen Lösungsmittels, beispielsweise eines niederen Alkanoles wie Methanol, kann hierbei zweckmäßig sein. Für die Umsetzung geeignete Temperaturen liegen zwischen -10 °C und 60 °C, bevorzugt zwischen 5 °C und Raumtemperatur. Als Reduktionsmittel eignen sich komplexe Hydride wie Alkalimetallborhydride, vorzugsweise Natriumborhydrid oder Natriumcyanoborhydrid. Ebenso kann die reduktive Alkylierung unter hydrogenolytischen Bedingungen durchgeführt werden. Die Hydrogenolyse kann unter den oben für die hydrogenolytische Abspaltung von Aminoschutzgruppen R¹⁰¹ aus Verbindungen der Formel I angegebenen Bedingungen durchgeführt werden. Verbindungen der Formel XVIIIb sind bekannt oder können nach an sich bekannten Methoden aus bekannten Verbindungen hergestellt werden.

Die zur Herstellung der Isocyanate der Formel VI geeigneten Amine der Formel VII können aus den 1-N-aminogeschützten Verbindungen der allgemeinen Formel XXII, 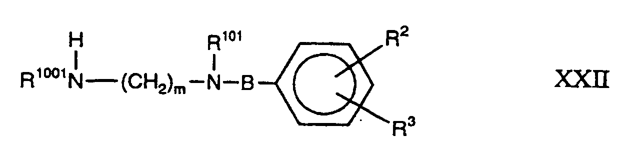 worin R¹⁰¹, R², R³, B und m obige Bedeutungen besitzen und R¹⁰¹ die für R⁹⁰¹ angegebene Bedeutung besitzt, erhalten werden, indem man aus Verbindungen der Formel XXII in an sich bekannter Weise die Aminoschutzgruppe R¹⁰⁰¹ selektiv unter Bedingungen abspaltet, unter denen die Schutzgruppe R¹⁰¹ nicht angegriffen wird. Beispielsweise kann die Schutzgruppe R¹⁰⁰¹ unter sauren Bedingungen abgespalten werden.

Verbindungen der Formel XXII können erhalten werden, indem man Amide der allgemeinen Formel XXIII, 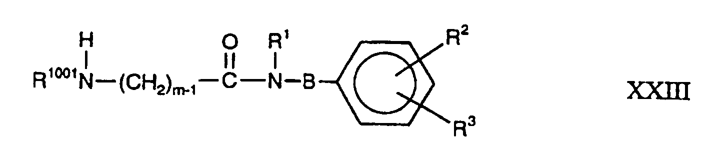 worin R¹, R², R³, R¹⁰⁰¹, B und m obige Bedeutungen besitzen, reduziert und anschließend in Verbindungen, worin R¹ für Wasserstoff steht, eine Aminoschutzgruppe R¹⁰¹ einführt. Die Reduktion kann mit komplexen Alkalimetallhydriden wie Lithiumaluminiumhydrid als Reduktionsmittel durchgeführt werden. Als Lösungsmittel eignen sich unter den Reaktionsbedingungen inerte organische Lösungsmittel wie niedere aliphatische Ether, beispielsweise Dioxan, THF oder Diethylether. Ein geeigneter Temperaturbereich liegt zwischen -20 °C und der Siedetemperatur des Reaktionsgemisches. Vorzugsweise kann die Reduktion bei Raumtemperatur durchgeführt werden.

Die Amide der Formel XXIII können durch Umsetzung von amino-geschützten ω-Aminocarbonsäuren der allgemeinen Formel XXIV, 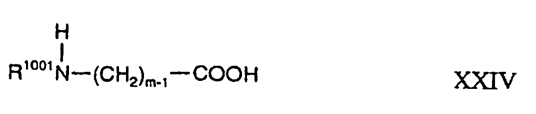 worin R¹⁰⁰¹ und m obige Bedeutungen besitzen, mit den Aminen der Formel IXa nach für die Amidbildung üblichen Methoden hergestellt werden. Beispielsweise kann die Amidbildung nach dem oben für die Umsetzung von Verbindungen der Formel IIa mit Verbindungen der Formel III beschriebenen Verfahren ausgeführt werden. Die Säuren der Formel XXIV stellen amino-geschützte ω-Aminocarbonsäuren dar, welche in ungeschützer Form allgemein bekannt sind und welche nach an sich bekannten Methoden aus den ungeschützten Vorläuferverbindungen hergestellt werden können.

Amine der Formel VII, worin m für die Zahl 3 steht, können insbesondere auch hergestellt werden, indem man die Cyanide der allgemeinen Formel XXV, 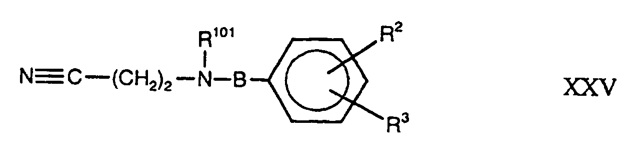 worin R¹⁰¹, R², R³ und B obige Bedeutungen besitzen, auf an sich bekannte Weise reduziert. Die Reduktion kann durch katalytische Hydrierung erfolgen, wobei sich Metall-Hydrierungskatalysatoren wie Raney-Nickel als Katalysatoren eignen. Geeignete Lösungsmittel sind unter den Reaktionsbedingungen inerte polare organische Lösungsmittel wie niedere Alkanole, beispielsweise Methanol oder Ethanol. Üblicherweise wird die Reaktion bei Raumtemperatur und bei einem Druck von 1 bis 3 bar, vorzugsweise von 2 bar durchgeführt. Zur Vermeidung von Nebenreaktionen kann der Reaktionslösung vor Zugabe des Katalysators eine ausreichende Menge einer konzentrierten wäßrigen Ammoniaklösung zugesetzt werden.

Die Cyanide der Formel XXV können durch Umsetzung von Acrylnitril der Formel XXVI, 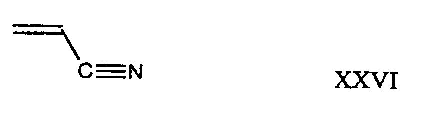 mit Aminen der Formel IXb hergestellt werden. Die Reaktion kann unter zur Durchführung von Michael-Additionen geeigneten, an sich bekannten Bedingungen durchgeführt werden. Als Lösungsmittel können unter den Reaktionsbedingungen inerte polare aprotische Lösungsmittel wie DMF, DMSO oder Dichlormethan verwendet werden. Üblicherweise wird die Umsetzung bei Temperaturen zwischen -20 °C und 60 °C, vorzugsweise bei Raumtemperatur ausgeführt. Zur Beschleunigung der Reaktion ist es vorteilhaft, dem Reaktionsgemisch einen geeigneten Katalysator zuzusetzen. Als Katalysatoren eignen sich starke Basen wie quartäre Alkyl- oder Phenylniederalkylammoniumhydroxide, beispielsweise Benzyltrimethylammoniumhydroxid.

Die Diazepane der Formel XIIa können auf an sich bekannte Weise, beispielsweise durch Reduktion der Diazepanone der allgemeinen Formel XXVIIa, 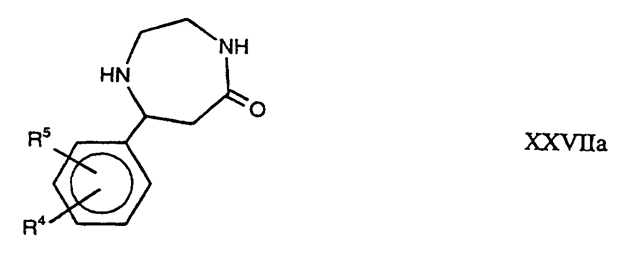 worin R⁴ und R⁵ obige Bedeutungen besitzen, erhalten werden. Die Reduktion kann nach dem oben für die Reduktion von Amiden der Formel XXIII angegebenen Verfahren durchgeführt werden.

Die Verbindungen der Formel XXVIIa sind teilweise bekannt aus C.H. Hofmann, S.R. Safir, Journal of Organic Chemistry 27 (1962), Seiten 3565 bis 3568, und können nach den dort beschriebenen oder dazu analogen Verfahren erhalten werden. Beispielsweise können die Diazepanone der Formel XXVIIa durch an sich bekannte katalytische Hydrierung der Diazepinone der allgemeinen Formel XXVIII, 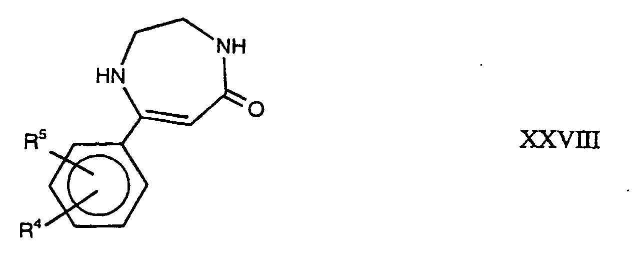 worin R⁴ und R⁵ obige Bedeutungen besitzen, hergestellt werden.

Die erhaltenen Diazepanone der Formel XXVIIa enthalten an dem die Phenylgruppe tragenden Kohlenstoffatom ein Chiralitätszentrum. Üblicherweise werden die Diazepanone der Formel XXVIIa bei der Herstellung als Racemate erhalten. Racemische Gemische von Verbindungen der Formel XXVIIa können auf an sich bekannte Weise in ihre optischen Isomere aufgetrennt werden, beispielsweise durch Umsetzung mit geeigneten optisch aktiven Säuren wie Campher-10-sulfonsäure und anschließender Auftrennung in ihre optisch aktiven Antipoden durch fraktionierte Kristallisation der gewonnenen diastereomeren Salze.

Die Verbindungen der Formel XXVIII können auf an sich bekannte Weise, wie zum Beispiel durch Kondensation von Ethylendiamin mit den Ethylbenzoylacetaten der allgemeinen Formel XXIX, 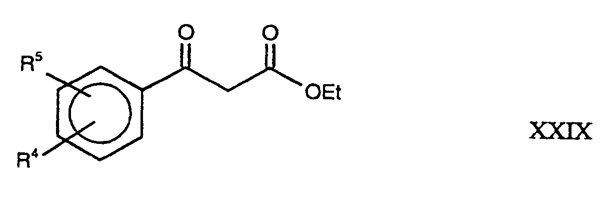 worin R⁴ und R⁵ die obigen Bedeutungen besitzen, hergestellt werden.

Die Verbindungen der Formel XIIb können aus den Diazepanon-Verbindungen der allgemeinen Formel XXVIIb, 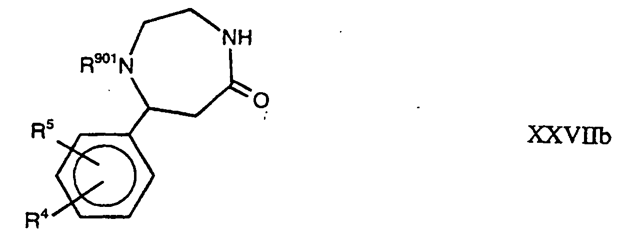 worin R⁴, R⁵ und R⁹⁰¹ obige Bedeutungen besitzen, auf an sich bekannte Weise durch Reduktion gewonnen werden. Die Reduktion kann beispielsweise nach der oben für die Reduktion von Amiden der Formel XXIII beschriebenen Methode durchgeführt werden. Die Verbindungen der Formel XXVIIb können durch Einführung geeigneter Schutzgruppen in Verbindungen der Formel XXVIIa gewonnen werden.

In der Synthese von Zwischenprodukten der Formel IIa eingesetzte Verbindungen der Formel XIII, worin A für eine -(CH₂)ₙ-Gruppe steht, worin n obige Bedeutung besitzt, können hergestellt werden, indem man Verbindungen der allgemeinen Formel XIIc, 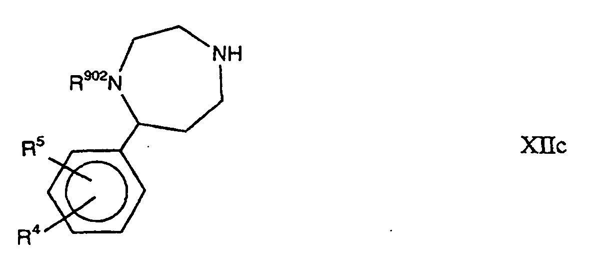 worin R⁴, R⁵ und R⁹⁰² obige Bedeutungen besitzen, mit Carbonsäuren der Formel XV unter an sich zur Aminoacylierung üblichen Bedingungen umsetzt. Insbesondere kann die Amidbildung nach dem oben für die Umsetzung von Verbindungen der Formel IIa mit Verbindungen der Formel III angegebenen Verfahren durchgeführt werden.

Verbindungen der Formel XIII, worin A für eine -NH (CH₂)ₘ-Gruppe steht, worin m obige Bedeutung besitzt, können hergestellt werden, indem man die Amine der Formel XIIc mit Isocyanaten der Formel XVI umsetzt. Die Umsetzung kann auf die oben für die Umsetzung von Aminen der Formel IV mit Isocyanaten der Formel VI beschriebene Weise durchgeführt werden.

Einfach amino-geschützte Diazepanderivate der Formel XIIc können auf an sich bekannte Weise durch Reduktion aus den einfach amino-geschützten Diazepanonen der allgemeinen Formel XXVIIc, 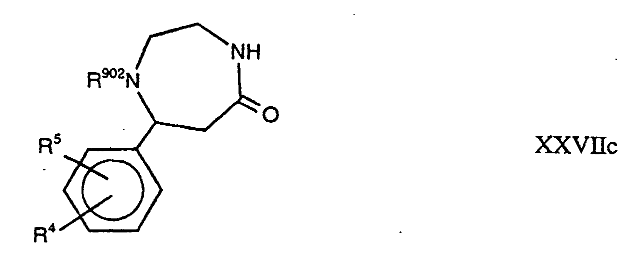 worin R⁴, R⁵ und R⁹⁰² obige Bedeutungen besitzen, erhalten werden. Die Reduktion kann analog zu dem oben für die Reduktion von Amiden der Formel XXIII angegebenen Verfahren erfolgen. Die amino-geschützten Diazepanone der Formel XXVIIc können durch an sich bekannte Einführung geeigneter AminoSchutzgruppen aus den Diazepanonen der Formel XXVIIa erhalten werden.

Die einfach aminogeschützten Diazepane der Formel XIIc können auch durch selektive Entfernung nur einer Aminoschutzgruppe aus Bis-aminogeschützten Diazepanen der allgemeinen Formel XIId, 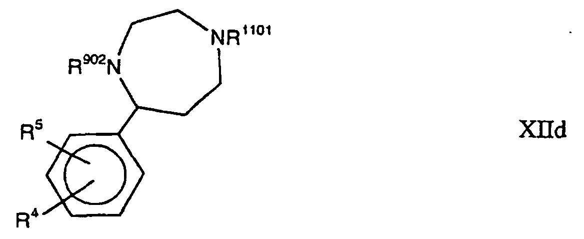 worin R⁴, R⁵ und R⁹⁰² obige Bedeutungen besitzen und R¹¹⁰¹ die für R⁹⁰² angegebene Bedeutung besitzt, erhalten werden. Sofern beispielsweise R⁹⁰² und R¹¹⁰¹ beide für die Benzylgruppe stehen, kann selektiv nur die Benzylgruppe R¹¹⁰¹ abgespalten werden, indem man Verbindungen der Formel XIId mit einem Chlorameisensäure-Derivat, beispielsweise dem Chlorameisensäure-1-chlorethylester, in einem unter den Reaktionsbedingungen inerten polaren aprotischen Lösungsmittel wie einem Niederalkylcyanid, beispielsweise Acetonitril, einem partiell halogenierten niederen Alkan, beispielsweise Dichlormethan, einem Diniederalkylether wie THF, Dioxan oder Diethylether oder anderen aprotischen Lösungsmitteln wie DMF oder DMSO, umsetzt und das entstandene Zwischenprodukt anschließend durch Zugabe eines geeigneten Reagenzes zum gewünschten Produkt spaltet. Sofern Chlorameisensäure-1-chlorethylester verwendet wird, eignen sich niedere Alkanole wie Methanol als Reagenzien zur Spaltung zum Produkt. Zweckmäßigerweise wählt man zu Beginn der Reaktion, während der Vermischung der Reaktanten, eine niedrigere Temperatur, beispielsweise zwischen - 20 °C und 10 °C, vorzugsweise zwischen -5 °C und 5 °C, gibt dann das zur Spaltung zum Produkt geeignete Reagenz, beispielsweise Methanol, zu und erhöht anschließend zur Vervollständigung der Reaktion die Temperatur auf 30 °C bis 70 °C, vorzugsweise auf 40 °C bis 50 °C. Zweckmäßigerweise kann vor der Zugabe des Reagenzes das Volumen der Reaktionsmischung, beispielsweise um ca. ein Drittel, auf an sich bekannte Weise vermindert werden.

Bis-aminogeschützte Diazepane der Formel XIId können beispielsweise hergestellt werden, indem man die Diamine der allgemeinen Formel XXX, 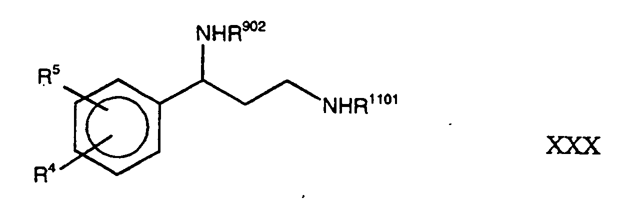 worin R⁴, R⁵, R⁹⁰² und R¹¹⁰¹ obige Bedeutungen besitzen, mit Glyoxal der Formel XXXI,

OHC-CHO XXXI

unter allgemein zur reduktiven Alkylierung von Aminen üblichen Bedingungen kondensiert. Als Reduktionsmittel eignen sich beispielsweise komplexe Alkalimetallborhydride wie Natriumcyanoborhydrid. Geeignete Lösungsmittel sind polare organische Lösungsmittel wie niedere Alkanole, beispielsweise Methanol oder Ethanol. Üblicherweise kann die Reaktion bei Temperaturen zwischen -20 °C und ca. 60 °C, vorzugsweise bei Raumtemperatur ausgeführt werden.

Diamine der Formel XXX, worin R⁹⁰² die Benzylgruppe darstellt, sind nach allgemein üblichen Verfahren, beispielsweise durch Reduktion mit komplexen Alkalimetallhydriden, aus den Verbindungen der allgemeinen Formel XXXII, 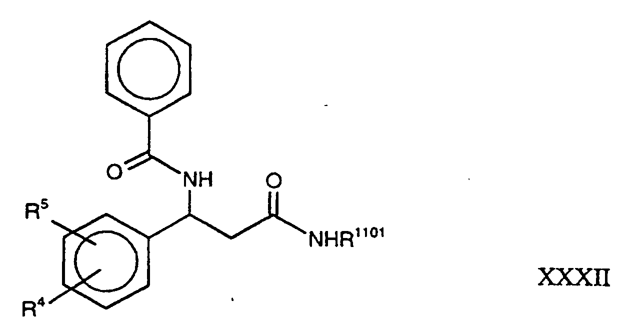 worin R⁴, R⁵ und R¹¹⁰¹ obige Bedeutungen besitzen, herstellbar. Die Reduktion kann zum Beispiel nach dem oben für die Reduktion von Amiden der Formel XXIII angegebenen Verfahren erfolgen. Bis-amino-geschützte Verbindungen der Formel XXX, worin die Aminoschutzgruppen R⁹⁰² und/oder R¹¹⁰¹ andere Bedeutungen als Benzylgruppen besitzen, können beispielsweise durch Einführung der gewünschten Schutzgruppen in geeignete Vorläuferverbindungen der Verbindungen der Formel XXX erhalten werden.

Die Verbindungen der Formel XXX enthalten ein chirales Kohlenstoffatom und können in Form zweier unterschiedlicher Enantiomere vorkommen. Sofern von reinen Ausgangsverbindungen, beispielsweise Verbindungen der Formel XXXIII, ausgegangen wird, entstehen auch reine Isomere von Verbindungen der Formel XXX.

Verbindungen der Formel XXXII können aus den Amino-acylierten 3-Amino-3-phenylpropionsäuren der allgemeinen Formel XXXIII, 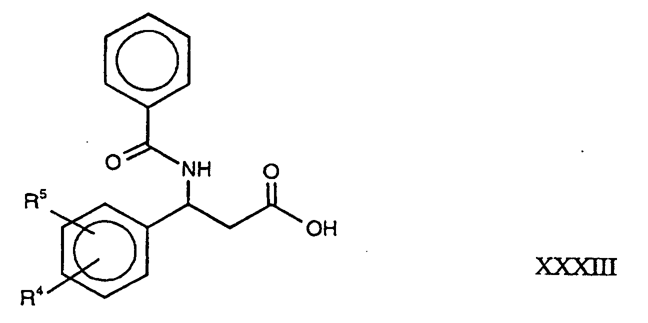 worin R⁴ und R⁵ obige Bedeutungen besitzen, durch Umsetzung mit den Aminen der allgemeinen Formel XXXIV,

H₂N-R¹¹⁰¹ XXXIV

worin R¹¹⁰¹ obige Bedeutung besitzt, hergestellt werden. Die Reaktion kann nach allgemein zur Amidbildung üblichen Bedingungen, beispielsweise nach dem oben für die Umsetzung von Verbindungen der Formel IIa mit Verbindungen der Formel III angegebenen Verfahren durchgeführt werden.

Die Verbindungen der Formel XXXIII können durch an sich bekannte Benzoylierung der Aminogruppe von 3-Amino-3-phenylpropionsäuren der allgemeinen Formel XXXV, 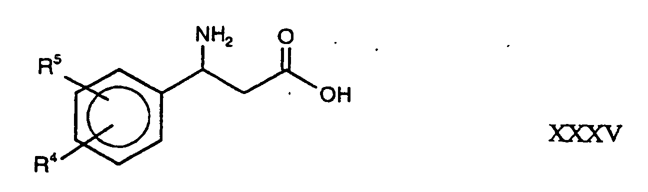 worin R⁴ und R⁵ obige Bedeutungen besitzen, hergestellt werden. Sofern Verbindungen der Formel XXX gewünscht sind, worin R⁹⁰² eine andere Aminoschutzgruppe als die Benzylgruppe ist, können diese anderen Aminoschutzgruppen oder deren Vorstufen, welche durch Reduktion mit komplexen Alkalimetallhydriden in die entsprechenden Aminoschutzgruppen überführt werden können, zweckmäßigerweise bereits in Amine der Formel XXXV eingeführt werden.

Die Verbindungen der Formel XXXIII enthalten ein chirales Kohlenstoffatom und können in Form zweier unterschiedlicher Enantiomere auftreten. Sofern bei der Herstellung von Verbindungen der Formel XXXIII racemische Gemische von Verbindungen der Formel XXXV eingesetzt werden, entstehen auch racemische Gemische von Verbindungen der Formel XXXIII. Zur Herstellung von reinen Enantiomeren der Verbindungen der Formel XXX kann zweckmäßigerweise von reinen Enantiomeren der Formel XXXIII ausgegangen werden. Reine Enantiomere der Verbindungen der Formel XXXIII können durch an sich bekannte Auftrennung ihrer racemischen Gemische erhalten werden. Die Auftrennung kann durch chromatographische Trennung an chiralen Trennmaterialien erfolgen oder durch Umsetzung mit geeigneten optisch aktiven Basen wie α-Methylbenzylamin und anschließender Auftrennung in die optisch aktiven Antipoden durch fraktionierte Kristallisation der gewonnenen diastereomeren Salze.

Verbindungen der Formel XXXV können durch an sich bekannte Kondensation von aromatischen Aldehyden der allgemeinen Formel XXXVI, 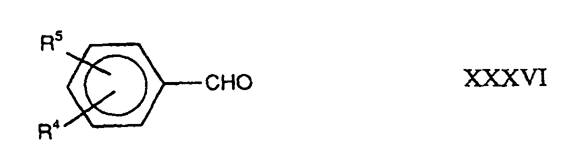 worin R⁴ und R⁵ obige Bedeutungen besitzen, mit Malonsäure der Formel XXXVII,

HOOC-CH₂-COOH XXXVII

oder deren Niederalkylester, und einem Ammoniumsalz, beispielsweise Ammoniumacetat, hergestellt werden. Die Reaktion kann in einem polaren protischen organischen Lösungsmittel wie einem niederen Alkanol, beispielsweise Methanol oder Ethanol, und bei Temperaturen zwischen Raumtemperatur und dem Siedepunkt des Reaktionsgemisches, vorzugsweise zwischen 70 °C und 90 °C, durchgeführt werden.

Verbindungen der Formel XXXV können auch aus α-Aminosäuren der allgemeinen Formel XXXVIII, 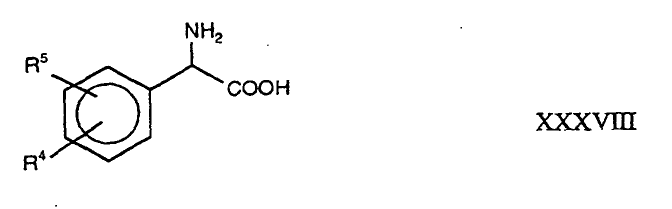 worin R⁴ und R⁵ obige Bedeutungen besitzen, auf an sich zur Kettenverlängerung von Carbonsäuren um eine Methylengruppe bekannte Weise hergestellt werden. Die Verlängerung um eine Methyleneinheit kann beispielsweise erfolgen, indem man die Carboxylgruppen von Verbindungen der Formel XXXVIII, beispielsweise durch Reduktion mit komplexen Metallhydriden wie Lithiumaluminiumhydrid, in Methylenhydroxygruppen überführt, und erhaltene Hydroxylgruppen auf an sich bekannte Weise in gute Abgangsgruppen wie Sulfonsäureester, zum Beispiel einen Trifluormethylsulfonsäureester verwandelt. Diese Abgangsgruppen können anschließend mit Alkalimetallcyaniden wie Natriumcyanid durch die Cyanogruppe substituiert werden, welche unter den hierfür üblichen Bedingungen zur Carboxylgruppe hydrolysiert werden kann, um Aminopropionsäuren der Formel XXXV zu erhalten. Sofern von optisch aktiven Aminosäuren der Formel XXXVIII ausgegangen wird, werden auch optisch aktive Aminosäuren der Formel XXXV erhalten.

Die zur Herstellung von Verbindungen der Formel IV verwendeten Ausgangsverbindungen der Formel XIV können durch Umsetzung von Verbindungen der Formel III mit Verbindungen der Formel XXVIIa nach der oben für die Umsetzung von Verbindungen der Formel IIa mit Verbindungen der Formel III angegebenen Methode hergestellt werden.

Verbindungen der Formel XIX können unter für Aminoacylierungen allgemein bekannten Bedingungen, beispielsweise nach der oben für die Umsetzung von Verbindungen der Formel IIa mit Verbindungen der Formel III angegebenen Methode durch Umsetzung der einfach amino-geschützten Diazepane der Formel XIIc mit den Carbonsäuren der Formel XVIIIa und nachfolgender selektiver Abspaltung der Amino-Schutzgruppe R⁹⁰² auf an sich bekannte Weise erhalten werden.

Durch die vorstehend angeführten Umsetzungen von chiralen Verbindungen der Formel XXVIIa, XXXIII, XXXV, XXXVIII oder von chiralen, ein- oder mehrfach durch niederes Alkyl substituierte Alkylenketten B oder B¹ enthaltenden Verbindungen treten an den jeweils darin enthaltenen Chiralitätszentren keine Veränderungen auf. Aus den optisch reinen Ausgangsverbindungen der Formel XXVIIa, XXXIII, XXXV, XXXVIII und aus durch niederes Alkyl substituierte Alkylenketten B oder B¹ enthaltenden Verbindungen enstehen daher auch optisch reine Folgeprodukte, insbesondere optisch reine Verbindungen der Formel I.

Die Verbindungen der allgemeinen Formel I und ihre physiologisch verträglichen Salze besitzen interessante pharmakologische Eigenschaften und zeichnen sich durch hohe Affinität zu Neurokininrezeptoren, vornehmlich NK-1-Rezeptoren, aus.

Aufgrund ihrer Neurokinin-Rezeptor-antagonistischen Eigenschaften eignen sich die Substanzen zur Behandlung von durch Neurokinine induzierten Krankheitszuständen. Beispielsweise eignen sich die Substanzen zur Hemmung von durch an NK-1-Rezeptoren bindende Neurokinine induzierte Vorgänge bei der Schmerzübertragung, Emese, neurogenen Entzündungen und asthmatischen Beschwerden. Dabei zeigen die Substanzen ein für die Behandlung von funktionellen und entzündlichen Störungen im gastrointestinalen Trakt sowie Übelkeit günstiges Wirkungsprofil. Zu den durch die erfindungsgemäßen Verbindungen behandelbaren funktionellen Störungen zählen insbesondere die als sogenanntes "irritable bowel syndrome" (= IBS) oder Reizdarmsyndrom bekannten Störungen der unteren Darmwege. Wesentliche Symptome von IBS sind Unterbauchschmerzen, die erheblich durch eine Übersensibilität des visceralen afferenten Nervensystems mitverursacht werden, und Anomalien des Stuhlganges, insbesondere anomal beschleunigte Passage des Stuhls im Colon. Die erhöhte viscerale Schmerzempfindlichkeit gegenüber mechanischen oder chemischen Reizen im intestinalen Trakt führt dazu, daß IBS-Patienten schon bei physiologischen verdauungsbedingten geringen Dehnungen des Colons, z. B. bereits bei geringer Gasbildung und leichten Blähungen, die von Gesunden kaum wahrgenommen werden, starke viscerale Schmerzen erleiden. An der Schmerzübertragung im gastrointestinalen Bereich sind an NK-1-Rezeptoren bindende Neurokinine als Neurotransmitter maßgeblich beteiligt. Die erfindungsgemäßen neurokininantagonistischen Substanzen weisen ein günstiges Wirkungsprofil mit ausgeprägten Wirkkomponenten gegenüber visceralen Schmerzen und Störungen der Stuhlpassage im Colon sowie Übelkeit auf. Zu durch die erfindungsgemäßen Verbindungen günstig beeinflußbaren inflammatorisch bedingten Störungen im gastrointestinalen Trakt gehören die im allgemeinen unter dem Begriff IBD (= inflammatory bowel disease) zusammengefaßten entzündlichen Störungen im Dünndarm- und Dickdarmbereich, u. a. Colitis ulcerosa und Morbus Crohn. Das Wirkprofil der Verbindungen zeichnet sich durch eine hohe Selektivität der gastrointestinalen und antiemetischen Wirksamkeit und eine gute Verträglichkeit mit einem günstigen Verhältnis von gastrointestinaler Wirksamkeit zu cardiovasculären calciumantagonistischen Nebenwirkungen sowie durch gute orale Wirksamkeit aus.

### Beschreibung der pharmakologischen Testmethoden

### 1. Bestimmung des Bindungsvermögens der Testsubstanzen an NK-1-Rezeptoren

Die Affinität der Testsubstanzen zu humanen NK-1-Rezeptoren wird in vitro gemessen. Bestimmt wird die Hemmung der Bindung der physiologischen Neurokinin-Substanz P an Neurokinin-1-Rezeptoren.

Die Rezeptor-Bindungsstudien werden mit [³H]-Substanz P als Ligand durchgeführt. Für den Bindungversuch werden verschiedenen Proben einer Membranpräparation von CHO-Zellen (= Eizellen des chinesischen Hamsters, chinese hamster oocytes), die den humanen NK-1-Rezeptor exprimieren, mit einer Lösung des markierten Liganden inkubiert, wobei die Inkubationsansätze keine Testsubstanz oder Zusätze unterschiedlicher Konzentrationen an Testsubstanz enthalten. Anschließend wird in den Proben jeweils eine Trennung von gebundenem und freiem Liganden mit Hilfe einer Glasfiber-Filtration vorgenommen. Die im Filter verbleibende Fraktion wird mehrmals mit Pufferlösung gewaschen und anschließend wird die Radioaktivität der im Filter verbliebenen Fraktion mit einem Beta-Scintillationszähler gemessen. Es wird als IC₅₀ der jeweiligen Testsubstanz diejenige Konzentration bestimmt, welche eine halbmaximale Verdrängung des gebundenen Liganden bewirkt. Aus dieser wird die entsprechende Inhibitionskonstante (Kᵢ-Wert) der Testsubstanz berechnet.

Die nachfolgende Tabelle 1 gibt nach der vorstehend beschriebenen Methode erhaltene Kᵢ-Werte für die Affinität der Testsubstanzen zu humanen NK-1-Rezeptoren wieder.

Die für die Verbindungen der Formel I in den nachfolgenden Tabellen zu den pharmakologischen Testmethoden angegebenen Beispielsnummern beziehen sich auf die nachstehenden Herstellungsbeispiele.

**Tabelle 1**

| **Bindungsaffinität zu humanen NK-1-Rezeptoren** | |
|---|---|
| Beispiel Nr. | in vitro Bindung an humane NK-1-Rezeptoren Kᵢ-Wert in µmol/l |
| 1 | Kᵢ = 0,012 |
| 2 | Kᵢ = 0,010 |
| 4 | Kᵢ = 0,008 |
| 14 | Kᵢ = 0,010 |

### 2. Untersuchung der Wirkung der Verbindungen auf die Stuhlpassage durch das Colon der Ratte

Die Wirkungen der Testsubstanzen auf den Transport des Stuhles durch den Dickdarm werden an Ratten nach Fütterung geprüft. Als Maß für die zur Stuhlausscheidung führende Co-lonmotilität werden nach Fütterung der Beginn der Ausscheidung und die mittlere Verweilzeit von Bariumsulfat im Colon bestimmt.

Nach oraler Gabe der Testsubstanz wird den Tieren 2 ml einer 80 % Bariumsulfatsuspension über einen künstlichen Ausgang am Blinddarm appliziert. Die Tiere werden in Stoffwechselkäfige gesetzt und die Faeces in 1-stündigen Intervallen über 24 h gesammelt. Der Gehalt an Bariumsulfat in den Faeces wird radiographisch gemessen und daraus der Beginn der Bariumsulfatausscheidung und die mittlere Retentionszeit bestimmt. Aus der nachfolgenden Tabelle 2 sind die mit verschiedenen Dosierungen der Testsubstanz des Beispiels 1 in dieser Versuchsanordnung erzielten Verzögerungen des Beginns der Ausscheidung und Verlängerungen der mittleren Verweilzeit des Bariumsulfates ersichtlich. Die Zeitspanne bis zum Beginn der Ausscheidung und die mittlere Retentionszeit werden in %-Werten bezogen auf die in einem Kontrollversuch ohne Testsubstanz erhaltenen Werte (= 100 %) angegeben.

**Tabelle 2**

| **Beeinflussung der Colonpassage** | | |
|---|---|---|
| Dosis der Testsubstanz des Beispiels 1 | Zeitspanne bis zum Beginn der Ausscheidung in % bezogen auf Kontrollwert = 100% | mittlere Retentionszeit in % bezogen auf Kontrollwert = 100% |
| 10 µmol/kg | 100 | 98 |
| 21.5 µmol/kg | 115 | 106 |
| 100 µmol/kg | 138 | 119 |

Die Testergebisse zeigen, daß die Testsubstanz die zur Kotausscheidung führende Colonaktivität dämpfen kann.

### 3. Untersuchung der Wirkung der Verbindungen auf die viscerale Schmerzempfindlichkeit an der Ratte

Visceraler Schmerz führt zu visceralen Reaktionen, die sich u.a. durch Kontraktionen der Bauchmuskulatur manifestieren. Die Anzahl der nach einem durch Dehnung des Dickdarms erzeugten mechanischen Schmerzreiz auftretenden Kontraktionen der Bauchmuskulatur ist somit ein Maß für die Bestimmung der visceralen Schmerzempfindlichkeit.

Die hemmende Wirkung der Testsubstanzen auf dehnungsinduzierte Bauchkontraktionen wird an Ratten geprüft. Die Dehnung des Dickdarms mit einem eingeführten Ballon wird als Reiz verwendet; als Antwort wird die Kontraktion der Bauchmuskulatur gemessen. Eine Stunde nach Sensibilisierung des Dickdarms durch Instillation von verdünnter Essigsäure (0.6 %, 1,5 ml) wird ein Latexballon eingeführt und für 10 min mit 100 mbar aufgeblasen. Während dieser Zeit werden die Kontraktionen der Bauchmuskulatur gezählt. 20 min nach subcutaner Applikation der Testsubstanz wird diese Messung wiederholt. Die Wirkung der Testsubstanz wird als prozentuale Abnahme der gezählten Kontraktionen im Vergleich zur Kontrolle errechnet. Die mit verschiedenen Dosierungen der Testsubstanz des Beispiels 1 erzielten Verringerungen der Anzahl der Bauchkontraktionen werden in der nachfolgenden Tabelle 3 in %-Werten, bezogen auf die vor der Substanzeinnahme gemessenen Kontrollwerte (= 100 %) angegeben.

**Tabelle 3**

| **Beeinflussung der visceralen Empfindlichkeit** | |
|---|---|
| Dosis der Testsubstanz des Beispiels 1 | Reduktion der Anzahl der dehnungsinduzierten Kontraktionen der Bauchmuskulatur in % bezogen auf Kontrollwert = 100% |
| 10 µmol/kg | 33 % |
| 21,5 µmol/kg | 39 % |
| 100 µmol/kg | 61 % |

Die durch die Testsubstanz erzielte Abnahme der Anzahl der durch Dehnungsreiz induzierten Bauchkontraktionen ist ein deutlicher Indikator für die Wirksamkeit der Testsubstanzen gegenüber visceraler Schmerzempfindlichkeit.

Die vorstehenden pharmakologischen Versuchsergebnisse zei-gen, daß die Verbindungen der Formel I die durch Stimulation der afferenten Nerven verursachten Störungen der Colonmotilität verhindern können und deshalb für die Behandlung von IBS geeignet sind. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größeren Säugetieren jedoch Arzneiformen mit einem Wirkstoffgehalt von 0,1 bis 80 mg, insbesondere 1 bis 10 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese Präparate können pharmazeutisch übliche anorganische und/oder organische Trägerstoffe, wie z. B. Talkum, Milchzucker oder Stärke neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden wie z. B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die nachfolgend angeführten Beispiele sollen die Erfindung näher erläutern, ohne sie in ihrem Umfang zu beschränken.

Die Strukturen der neuen Verbindungen wurden zum Teil durch spektroskopische Untersuchungen, insbesondere durch Analyse der Massen- oder IR-Spektren und gegebenenfalls durch Bestimmung der optischen Drehwerte gesichert.

### Beispiel 1:

### 1-(3,5-Bistrifluormethylbenzoyl)-4-{[3-[N-(2-methoxybenzyl)-N-methyl]amino]propylcarbonyl}-7-phenyl-1,4-diazepan

A) 10,3 g 4-Aminobuttersäure in 300 ml Methanol wurden mit 13,5 g 2-Methoxybenzaldehyd und 3,5 ml Triethylamin versetzt und in Gegenwart von 10 g Raney-Nickel bei 2 bar hydriert. Nach vollendetem Umsatz wurden 12,6 ml einer 37%igen wäßrigen Formaldehydlösung und 11,4 ml Triethylamin zugegeben. Nach Zugabe von weiteren 5 g Raney-Nickel wurde erneut bis zur Beendigung der Wasserstoffaufnahme hydriert. Anschließend wurde das Gemisch vom Katalysator abfiltriert und im Vakuum eingeengt. Das als Rohprodukt erhaltene [N-Methyl-N-(2-methoxybenzyl)]-4-aminobuttersäure-Triethylaminsalz wurde ohne weitere Reinigung für die nächste Synthesestufe eingesetzt.
B) 96 g Benzoylessigsäureethylester in 200 ml Pyridin wurden mit 30 g Ethylendiamin versetzt und fünf Stunden lang unter Rückfluß gekocht. Anschließend wurde das Pyridin abdestilliert und der Rückstand eine Stunde lang auf 180 °C erhitzt. Nach Abkühlung wurden 500 ml Dichlormethan zugegeben und die Reaktionsmischung bis zur Lösung auf 40 °C erwärmt. Ausfällung mit Aceton lieferte 21,7 g 7-Phenyl-1,2,3,4-tetrahydro-1,4-diazepin-5-on vom Fp. = 206 - 214 °C.
C) 70 g des oben erhaltenen Diazepinons in 300 ml Methanol wurden mit 8,0 g 10%igem Palladiumkatalysator auf Aktivkohle, aufgeschlämmt in 100 ml Ethanol, versetzt und dann bei 5 bar in einer Schüttelapparatur hydriert. Nach Beendigung der Wasserstoffaufnahme wurde vom Katalysator abfiltriert und das Filtrat im Vakuum zur Trockene eingeengt. Der Rückstand wurde in Ether aufgenommen, bis zur Trübung mit n-Hexan versetzt und im Kühlschrank über Nacht stehengelassen. Die ausgefallenen Kristalle wurden abfiltriert und die Mutterlauge zur Vervollständigung der Kristallisation im Vakuum eingeengt und mit n-Hexan versetzt. Waschen der vereinigten Feststofffraktionen mit n-Hexan und Trocknung lieferten 70,3 g Hexahydro-7-phenyl-1,4-diazepin-5-on vom Fp. = 85 - 86 °C.
D) 2,0 g LiAlH₄ wurden unter Stickstoffatomsphäre in THF suspendiert und dann bei 10 °C mit 5,0 g Hexahydro-7-Phenyl-1,4-diazepin-5-on versetzt. Anschließend wurde die Mischung 8 Stunden lang bei Raumtemperatur gerührt. Unter Stickstoffatmosphäre wurden dann nacheinander tropfenweise 5 ml Wasser, 2,2 g NaOH, gelöst in 5 ml Wasser und wieder 3 ml Wasser zugegeben und die Mischung 15 min bei Raumtemperatur gerührt. Nach Abfiltrieren der ausgefallenen Salze wurde das Filtrat im Vakuum zur Trockene eingeengt. Man erhielt 4,9 g 7-Phenyl-1,4-diazepan, das ohne weitere Reinigung umgesetzt wurde.
E) Zu einer Lösung von 4,8 g des oben unter A) erhaltenen rohen Triethylaminsalzes in 50 ml Dichlormethan wurden nacheinander 2,2 g Hydroxybenzotriazol in 50 ml DMF, 2,7 g Diisopropylcarbodiimid und 2,49 g des oben unter D) erhaltenen Diazepans in 20 ml Dichlormethan zugegeben und über Nacht bei Raumtemperatur gerührt. Das Lösungsmittel wurde im Vakuum abdestilliert und der Rückstand in 100 ml Dichlormethan aufgenommen. Nach Zugabe von 50 ml einer 10%igen wäßrigen Weinsäurelösung wurde geschüttelt und die organische Phase verworfen. Die wäßrige Phase wurde mit NaOH alkalisiert und 3 mal mit Dichlormethan extrahiert. Die vereinigten organischen Phasen wurden über Natriumsulfat getrocknet, filtriert und im Vakuum eingeengt. Man erhielt 5,8 g 4-{[[N-(2-Methoxybenzyl)-N-methyl]-amino]propylcarbonyl}-7-phenyl-1,4-diazepan als Rohprodukt, welches ohne Reinigung weiter umgesetzt wurde.
F) 5,8 g des oben erhaltenen Diazepans wurden in 50 ml Dichlormethan gelöst, bei 0 °C nacheinander mit 3,0 g Triethylamin und 4,06 g 3,5-Bis-(trifluormethyl)benzoylchlorid versetzt und 8 Stunden lang bei Raumtemperatur gerührt. Die Lösung wurde je einmal mit 10%iger wäßriger Weinsäurelösung und anschließend einmal mit 10%iger wäßriger Natronlauge ausgeschüttelt. Die vereinigten organischen Phasen wurden über Natriumsulfat getrocknet, filtriert und im Vakuum eingeengt. Anschließend wurde das Rohprodukt durch Chromatographie an Kieselgel (Laufmittel: Dichlormethan/Methanol) gereinigt. Man erhielt 4,5 g der Titelverbindung, welche mittels methanolischer HCl-Lösung in das Hydrochlorid überführt wurde. Ausbeute: 4,08 g; IR: 3030, 2940, 1635 cm⁻¹ (KBr); M⁺: 635.

### Beispiel 2:

### (-)-1-(3,5-Bistrifluormethylbenzoyl)-4-{[3-[N-(2-methoxybenzyl)-N-methyl]amino]propylcarbonyl}-7-phenyl-1,4-diazepan

A) 178,2 g Hexahydro-7-phenyl-1,4-diazepin-5-on (Herstellung siehe Beispiel 1C)) wurden in 400 ml Methanol und 200 ml Isopropanol gelöst, mit einer Lösung von 108,7 g (1S)-(+)-Campher-10-sulfonsäure in 800 ml Isopropanol bei 60 °C versetzt und das Gemisch zur Kristallisation über Nacht bei Raumtemperatur stehengelassen. Die überstehende Lösung wurde dekantiert und die Kristalle zweimal mit je 100 ml Isopropanol gewaschen. Zur Umkristallisation wurden die Kristalle bei 60 °C in 500 ml Methanol gelöst und anschließend mit 600 ml Isopropanol versetzt. Es wurde über Nacht stehengelassen und die Mutterlauge abdekantiert. Die Umkristallisation wurde insgesamt siebenmal wiederholt, wobei ab der 5. Umkristallisation die Lösungsmittelmengen auf jeweils 300 ml Methanol und 500 ml Isopropanol verringert wurden. Man erhielt 26,2 g des Camphersulfonats der Ausgangsverbindung mit einem optischen Drehwert [α]_{D}²⁰ = + 48,1° (c = 1,0 in MeOH). Das Camphersulfonat wurde in 300 ml Wasser gelöst und mit 10%iger Natronlauge auf pH 10 eingestellt. Nach Zugabe von ca 50 g Kochsalz wurde mit Dichlormethan extrahiert. Die vereinigten organischen Phasen wurden über Natriumsulfat getrocknet, filtriert und im Vakuum eingeengt. Man erhielt 9,8 g (+)-Hexahydro-7-phenyl-1,4-diazepin-5-on, [α]_{D}²⁰ = +13,1° (c = 1,0 in Me-OH).
B) 9,0 g des oben erhaltenen (+)-Diazepinons wurden mit 3,6 g LiAlH₄ analog zu der in Beispiel 1D) angegebenen Methode umgesetzt. Man erhielt 8,7 g 7-Phenyl-1,4-diazepan, welches ohne Reinigung weiter umgesetzt wurde. Ein kleiner Teil des Diazepans wurde zur Bestimmung des optischen Drehwertes durch Behandlung mit einer Lösung von HCl in Toluol in das Hydrochlorid überführt, [α]_{D}²⁰ = -46,7° (c = 1,0 in MeOH).
C) 8,7 g des vorstehend erhaltenen Diazepans wurden mit 16,8 g [N-Methyl-N-(2-methoxybenzyl)]-4-aminobuttersäure-Triethylaminsalz (Herstellung siehe Beispiel 1A)) nach der in Beispiel 1E) angegebenen Methode umgesetzt. Man erhielt 20,8 g eines Enantiomers des 4-{[[N-(2-Methoxybenzyl)-N-methyl]-amino]propylcarbonyl}-7-phenyl-1,4-diazepans als Rohprodukt, welches ohne weitere Reinigung umgesetzt wurde.
D) 20,8 g des oben erhaltenen enantiomerenreinen Diazepans wurden nach der in Beispiel 1F) angegebenen Methode mit 14,6 g 3,5-Bistrifluormethylbenzoylchlorid umgesetzt. Man erhielt 9,9 g der Titelverbindung mit dem optischen Drehwert [α]_{D}²⁰ = -33,7° (c = 1,0 in MeOH). Durch Zugabe von HCl in Toluol wurde das kristalline Hydrochlorid der Titelverbindung erhalten, Fp.= 96 - 101 °C, [α]_{D}²⁰ = -36,5° (c = 1,0 in MeOH).

### Beispiel 3:

### 1-(3,5-Bistrifluormethylbenzoyl)-4-{[[N-(2-phenylethyl)-N-methyl]amino]acetyl}-7-(2-fluorphenyl)-1,4-diazepan

A) 30,0 g 2-Fluorbenzaldehyd in 250 ml Ethanol wurden mit 25,2 g Malonsäure und 37,3 g Ammoniumacetat versetzt und 8 Stunden lang unter Rückfluß zum Sieden erhitzt. Nach Abkühlung des Gemisches wurden die Kristalle abfiltriert, zuerst mit Ethanol und anschließend mit einem Gemisch aus Ethanol und Wasser (75 : 25 v/v)gewaschen und bei 65 °C im Vakuum getrocknet. Man erhielt 16,5 g DL-3-Amino-3-(2-fluorphenyl)-aminopropionsäure, Fp. = 229 - 231 °C.
B) 10,0 g der oben erhaltenen Propionsäure wurden in einem Gemisch aus 200 ml THF und 50 ml Wasser gelöst und unter Eiskühlung tropfenweise abwechselnd mit 7,7 g Benzoylchlorid in 30 ml THF und 10%iger Natronlauge versetzt, so daß der pH-Wert bei ca. 10 gehalten wurde. Nach Beendigung der Zugabe wurde etwa 15 min bei Raumtemperatur gerührt. Anschließend wurde das Lösungsmittel im Vakuum abdestilliert und die verbleibende wäßrige Phase mit verdünnter wäßriger Salzsäurelösung auf pH 1 gebracht. Die ausgefallenen Kristalle wurden abfiltriert, mit Aceton gewaschen und im Vakuum getrocknet. Man erhielt 15,4 g N-Benzoyl-3-amino-3-(2-fluorphenyl)-propionsäure vom Fp. = 202 - 205 °C.
C) 10,0 g der oben erhaltenen benzoylierten Propionsäure wurden in 100 ml Dichlormethan gelöst, mit 5,8 ml Triethylamin versetzt und dann auf -10 °C abgekühlt. Anschließend wurden 3,32 ml Chlorameisensäureethylester tropfenweise hinzugegeben und das Reaktionsgemisch wurde weitere 30 min lang bei -10 °C gerührt. Dann wurden 3,81 ml Benzylamin tropfenweise zugegeben, und die Lösung wurde eine Stunde lang bei Raumtemperatur gerührt. Das Lösungsmittel wurde im Vakuum abgezogen und der Rückstand in Essigsäureethylester und Wasser aufgenommen und geschüttelt. Die organische Phase wurde im Vakuum eingeengt und das Gemisch anschließend zur Kristallisation stehengelassen. Die ausgefallenen Kristalle wurden mit Aceton gewaschen und im Vakuum getrocknet. Man erhielt 10,6 g N-Benzoyl-3-amino-3-(2-fluorphenyl)-propionsäure-benzylamid, Fp. = 223 - 226 °C.
D) 5,0 g LiAlH₄ wurden in einem Gemisch aus Toluol und THF (70 : 30 v/v) suspendiert und unter einer Schutzgasatmosphäre portionsweise mit 10,5 g des oben erhaltenen Benzylamids versetzt. Anschließend wurde die Mischung 8 Stunden lang unter Rückfluß gekocht, auf 0 °C abgekühlt und unter Stickstoff tropfenweise der Reihe nach mit 20 ml THF, 10 ml Wasser und 50 ml 10%iger Natronlauge versetzt. Der Ansatz wurde filtriert, die abgetrennten Salze mit Ethanol gewaschen und die vereinigten flüssigen Phasen im Vakuum zur Trockene eingedampft. Der Rückstand wurde in wenig Dichlormethan aufgenommen und mit Dichlormethan über Magnesiumsilikat (zur Chromatographie) filtriert. Das als Öl erhaltene Produkt wurde zur Salzbildung in 50 ml Dichlormethan gelöst, mit einem Überschuß an in Isopropanol gelöster Salzsäure versetzt und durch Zugabe von Diethylether auskristallisiert. Die Kristalle wurden abfiltriert und mit Diethylether gewaschen. Man erhielt 8,96 g 1-(2-Fluorphenyl)-1,3-(N,N'-dibenzyl)-diaminopropan als Dihydrochlorid vom Fp. = 195 - 198 °C.
E) 8,96 g des oben erhaltenen Dihydrochlorids in 70 ml Methanol wurden bei 10 °C mit 3,09 g 40%iger wäßriger Glyoxallösung und anschließend mit 4,68 g Natriumcyanoborhydrid portionsweise versetzt und das Gemisch 18 Stunden lang bei Raumtemperatur gerührt. Anschließend wurde die Mischung im Vakuum eingedampft, der Rückstand in Dichlormethan und Ethanol (90:10 v/v) aufgenommen und zuerst in diesem Lösungsmittelgemisch als Laufmittel über Kieselgel, anschließend in Dichlormethan/n-Hexan als Laufmittel über Aluminiumoxid gereinigt. Man erhielt 3,91 g 7-(2-Fluorphenyl)-(N,N'-dibenzyl)-1,4-diazepan, welches aus Ether/n-Hexan umkristallisiert wurde, Fp. = 82 - 83 °C.
F) 10,0 g des oben erhaltenen Diazepans in 50 ml 1,2-Dichlorethan wurden bei 0 °C tropfenweise mit 4,03 g Chlorameisensäure-1-chlorethylester versetzt und anschließend 2 Stunden lang unter Rückfluß gekocht. Das Gemisch wurde im Vakuum auf ca. 1/3 seines Volumens eingeengt, mit 30 ml Methanol versetzt und erneut 3 Stunden lang unter Rückfluß zum Sieden erhitzt. Dann wurde das Gemisch im Vakuum zur Trockene eingedampft, der Rückstand in 10 ml Dichlormethan aufgenommen und über Kieselgel chromatographiert (Laufmittel: Dichlormethan/Methanol). Es wurden 5,89 g 1-Benzyl-7-(2-fluorphenyl)-1,4-diazepan erhalten, welches ohne weitere Reinigung für die nächste Synthesestufe verwendet wurde.
   3,10 g des oben erhaltenen Monobenzyldiazepans in 20 ml Dichlormethan wurden bei 0 °C nacheinander mit 2,3 ml Diisopropylethylamin und 0,87 ml Chloracetylchlorid versetzt und 3 Stunden lang gerührt. Dann wurde die Lösung über Kieselgel chromatographiert (Laufmittel: Dichlormethan/Methanol 98:2). Nach Abdampfen des Lösungsmittels erhielt man 2,44 g 1-Benzyl-4-chloracetyl-7-(2-fluorphenyl-1,4)-diazepan als Öl, welches ohne weitere Reinigung und Charakterisierung für die nächste Synthesestufe eingesetzt wurde.
H) 1,43 g des oben erhaltenen 4-Chloracetyl-1,4-diazepans in 20 ml Methanol wurden mit 0,85 ml Diisopropylethylamin und 0,54 g N-Methylphenylethylamin versetzt und anschließend 5 Stunden lang unter Rückfluß zum Sieden erhitzt. Dann wurde das Gemisch im Vakuum eingedampft, der Rückstand in 5 ml Dichlormethan aufgenommen und durch Chromatographie an Kieselgel (Laufmittel Dichlormethan/ Methanol) gereinigt. Anschließend löste man das isolierte Rohprodukt in 20 ml Diethylether und versetzte es mit einem Überschuß methanolischer Salzsäurelösung. Das Gemisch wurde eingeengt und der Rückstand in wenig Dichlormethan aufgenommen. Nach Zugabe einiger Tropfen einer Lösung von Salzsäure in einem Gemisch aus Isopropanol und Diethylether kristallisierte das 1-Benzyl-4-{[[N-Phenylethyl-N-methyl]amino]acetyl}-7-(2-fluorphenyl)-1,4-diazepan als Hydrochlorid aus. Man erhielt 0,26 g Kristalle, Fp.= 141 - 143 °C.
I) 0,95 g der oben erhaltenen Diazepanverbindung in 20 ml Ethanol wurden mit 2 ml 2- normaler wäßriger Salzsäurelösung und 0,3 g 5%igem Palladiumkatalysator auf Aktivkohle versetzt und 5 Stunden lang bei Raumtemperatur hydriert. Dann wurde vom Katalysator abfiltriert und die Lösung im Vakuum eingedampft. Anschließend gab man 2 ml 10%ige Natronlauge und 30 ml Dichlormethan zu und schüttelte das Gemisch. Die organische Phase wurde abgetrennt, über Natriumsulfat getrocknet, im Vakuum auf ca. 10 ml eingeengt und über Kieselgel chromatographiert (Laufmittel: Dichlormethan/Methanol). Man erhielt 0,52 g 4-{[[N-Phenylethyl-N-methyl]amino]acetyl}-7-(2-Fluorphenyl)-1,4-diazepan als Öl, welches ohne weitere Reinigung und Charakterisierung für die nächste Synthesestufe eingesetzt wurde.
J) 0,52 g der oben erhaltenen öligen debenzylierten Diazepanverbindung wurden in der in Beispiel 1F) angegebenen Weise mit 0,75 g Diisopropylethylamin und 0,39 g 3,5 Bis-(trifluormethyl)benzoylchlorid umgesetzt. Man erhielt 0,82 g öliges Rohprodukt, welches in 50 ml Diethylether aufgenommen und mit einer Lösung von 0,16 g Maleinsäure in 5 ml THF versetzt wurde. Das entstandene Gemisch wurde auf ca. 10 ml eingeengt und im Kühlschrank zur Kristallisation gebracht. Man erhielt 0,7 g der Titelverbindung als Maleinat vom Fp.= 156 - 158 °C.

### Beispiel 4:

### 1-(3,5-Dimethylbenzoyl)-4-{[N-(2-methoxybenzyl)amino]acetyl}-7-phenyl-1,4-diazepan

A) 1,9 g Hexahydro-7-phenyl-1,4-diazepin-5-on (Herstellung siehe Beispiel 1C) in 30 ml Acetonitril wurden mit 2,0 g Kaliumcarbonat und 1,2 g Benzylchlorid unter Rückflußkühlung 36 Stunden lang zum Sieden erhitzt. Anschließend wurde filtriert und das Filtrat eingeengt. Der verbleibende Rückstand wurde mit 10%iger wäßriger Zitronensäurelösung geschüttelt, die organische Phase abgetrennt, über Magnesiumsulfat getrocknet und im Vakuum eingeengt. Man erhielt 2,23 g rohes 1-Benzyl-hexahydro-7-phenyl-1,4-diazepin-5-on als Öl, welches ohne weitere Reinigung und Charakterisierung für die nächste Synthesestufe eingesetzt wurde.
B) 2,2 g des oben erhaltenen benzylierten Diazepinons wurden nach der in Beispiel 3D) beschriebenen Weise mit 0,8 g LiAlH₄ reduziert. Man erhielt 1,64 g 1-Benzyl-7-phenyl-1,4-diazepan als Öl, welches ohne weitere Reinigung und Charakterisierung umgesetzt wurde.
C) 1,46 g der oben erhaltenen benzylierten Diazepanverbindung, 1,10 g N-(3-Dimethylaminopropyl)-N'-ethylcarbodiimid und 0,96 g tert.-Butyloxycarbonylglycin wurden in 30 ml Dichlormethan gelöst und 5 Stunden lang bei Raumtemperatur gerührt. Das Gemisch wurde mit 10%iger wäßriger Zitronensäurelösung geschüttelt, dann die organische Phase abgetrennt und über Magnesiumsulfat getrocknet. Einengen im Vakuum ergab 2,76 g rohes 1-Benzyl-4-{[N-(tert.-butyloxycarbonyl)aminolacetyl}-7-phenyl-1,4-diazepan, welches ohne weitere Reinigung umgesetzt wurde.
D) 2,5 g des oben erhaltenen monoacylierten Diazepanderivats in 100 ml Ethanol wurden mit 1,0 g 20%igem Palladiumhydroxydkatalysator auf Aktivkohle versetzt und 4 Stunden lang hydriert. Nach Abfiltrieren vom Katalysator wurde das Filtrat eingeengt, mit 15%iger wäßriger Weinsäurelösung geschüttelt und die wäßrige Phase mit 10%iger wäßriger Natronlauge alkalisch extrahiert. Abtrennung der organischen Phase, Trocknung über Magnesiumsulfat und Konzentration im Vakuum lieferte 1,78 g 4-[N-(tert.-Butyloxycarbonyl)amino]acetyl}-7-phenyl-1,4-diazepan, IR: 1700 cm⁻¹.
E) 1,7 g des oben erhaltenen debenzylierten Diazepans wurden nach der in Beispiel 1F) beschriebenen Weise mit 1,0 g Triethylamin und 0,86 g 3,5-Dimethylbenzoylchlorid umgesetzt. Man erhielt 2,42 g 1-(3,5-Dimethylbenzoyl)-4-{[N-tert.-butyloxycarbonyl]amino]acetyl}-7-phenyl-1,4-diazepan als Schaumharz, welches ohne weitere Reinigung und Charakterisierung umgesetzt wurde.
F) 2,08 g des oben erhaltenen bisacylierten Diazepans in 100 ml Dichlormethan wurden mit 10 ml Trifluoressigsäure versetzt und über Nacht bei Raumtemperatur gerührt. Anschließend wurde das Gemisch im Vakuum eingeengt, und der Rückstand wurde in 100 ml Dichlormethan aufgenommen und 3 mal mit 1- normaler Natronlauge geschüttelt. Die organische Phase wurde abgetrennt, über Natriumsulfat getrocknet und im Vakuum eingeengt. Man erhielt 1,5 g rohes 1-(3,5-Dimethylbenzoyl)-4-aminoacetyl-1,4-diazepan, welches ohne weitere Reinigung und Charakterisierung umgesetzt wurde.
G) 0,8 g der oben hergestellten Diazepanverbindung wurden in 100 ml Ethanol gelöst und nacheinander mit 0,3 g 2-Methoxybenzaldehyd und einer Spatelspitze Raney-Nickel versetzt. Dann wurde bei 3 bar und Raumtemperatur hydriert. Nach Beendigung der Wasserstoffaufnahme wurde vom Katalysator abfiltriert und das Filtrat im Vakuum konzentriert. Der Rückstand wurde an Kieselgel chromatographiert (Laufmittel: Dichlormethan/Methanol), wobei man 0,3 g der Titelverbindung erhielt,; IR: 3030, 2940, 1635 cm⁻¹; M⁺: 485.

### Beispiel 5:

### 1-(3,5-Bistrifluormethyl-benzoyl)-4-{[3-[N-(3-phenylpropyl)amino]propyl]amino-carbonyl}-7-phenyl-1,4-diazepan

A) 1,78 g β-Alanin in 30 ml THF wurden mit 0,8 g Natriumhydroxid, 10 ml Wasser und 4,36 g Di-tert.-butyldicarbonat versetzt und 60 Stunden lang bei Raumtemperatur gerührt. Anschließend wurde im Vakuum eingeengt, der Rückstand in Dichlormethan aufgenommen und mit 10%iger wäßriger Weinsäurelösung geschüttelt. Die organische Phase wurde abgetrennt, über Natriumsulfat getrocknet, filtriert und im Vakuum konzentriert. Man erhielt 3,35 g rohes N-tert.-Bu-Butyloxycarbonyl-β-Alanin, welches ohne Reinigung weiter umgesetzt wurde.
B) 3,0 g des oben hergestellten N-geschützten β-Alanins in 30 ml Dichlormethan wurden mit 3,5 g Triethylamin, 4,8 g 2-Chlor-1-methyl-pyridiniumjodid und 2,13 g 3-Phenyl-1-aminopropan versetzt und 18 Stunden lang bei Raumtemperatur gerührt. Anschließend wurde das Reaktionsgemisch mit 15%iger wäßriger Weinsäurelösung geschüttelt, dann die organische Phase abgetrennt und über Natriumsulfat getrocknet. Nach Filtrierung und Einengen wurde an Kieselgel chromatographiert (Laufmittel Dichlormethan/Methanol 99:1). Man erhielt 3,41 g N-tert.-Butyloxycarbonyl-C-[(3-phenylpropyl)amino]-β-Alanin, welches ungereinigt weiter umgesetzt wurde.
C) 23,7 g der oben hergestellten BOC-geschützten Verbindung wurden mit 8,0 g LiAlH₄ nach der in Beispiel 3D) beschriebenen Methode reduziert. Man erhielt 17,7 g öliges 1-[N-(tert.-Butyloxycarbonyl)amino]-3-[N-(3-phenylpropyl)-amino]-1,3-diaminopropan, welches ohne Reinigung weiter umgesetzt wurde.
D) 3,76 g des oben erhaltenen Diaminopropanderivates wurden in 50 ml THF gelöst und bei 10 °C abwechselnd portionsweise mit insgesamt 13 ml 1N Natronlauge und 2,4 g Benzyloxycarbonylchlorid versetzt. Nach vollständiger Umsetzung wurde die wäßrige Phase abgetrennt und 2 mal mit je 50 ml Dichlormethan extrahiert. Die vereinigten organischen Phasen wurden über Natriumsulfat getrocknet und im Vakuum eingeengt. Chromatographie an Kieselgel (Laufmittel: Dichlormethan/Methanol) lieferte 1-[N-tert.-Butoxycarbonyl-amino]-3-{[N-(3-phenylpropyl)-N-benzyloxycarbonyl)-amino}-1,3-diaminopropan als Zwischenprodukt, welches in 70 ml Acetonitril gelöst und mit 2,4 g p-Toluolsulfonsäure versetzt wurde. Man ließ dieses Gemisch 18 Stunden lang bei Raumtemperatur rühren und engte anschließend im Vakuum ein. Der Rückstand wurde mit 20 ml 1-normaler Natronlauge versetzt und 3 mal mit je 30 ml Dichlormethan extrahiert. Die vereinigten organischen Phasen wurden über Natriumsulfat getrocknet, filtriert und im Vakuum eingeengt. Chromatographie des Rückstandes an Kieselgel (Laufmittel: Dichlormethan/Methanol) lieferte 3,4 g öliges 1-Amino-3-[N-(3-phenylpropyl)-N-benzyloxycarbonyl]amino-1,3-diaminopropan, welches ohne Reinigung weiter umgesetzt wurde.
E) 16,4 g Hexahydro-7-phenyl-1,4-diazepan-5-on (Herstellung siehe Beispiel 1C)) in 100 ml THF wurden nach der oben unter A) beschriebenen Methode mit 25,5 ml 3-normaler Natronlauge und 19,0 g Di-tert.-butyldicarbonat umgesetzt. Man erhielt 23,4 g 1-tert.-Butyloxycarbonyl-hexahydro-7-phenyl-1,4-diazepin-5-on, welches ohne Reinigung weiter umgesetzt wurde.
F) 27,0 g des oben hergestellten BOC-geschützten Diazepinons wurden in THF gelöst und nach der oben in Beispiel 3 D) beschriebenen Methode mit 14,0 g LiAlH₄ 18 Stunden lang bei Raumtemperatur gerührt. Nach Abfiltrieren von den ausgefallenen Salzen wurde das Filtrat im Vakuum eingeengt und 2 mal über Kieselgel chromatographiert (Laufmittel: THF/MeOH). Man erhielt 6,5 g 1-tert.-Butyloxycarbonyl-7-phenyl-1,4-diazepan, welches ohne Reinigung weiter umgesetzt wurde.
G) 1,77 g des oben unter D) erhaltenen geschützten Diaminopropanderivates wurden in 50 ml Dichlormethan gelöst und unter Rühren und Eiskühlung mit 2,1 g Diisopropylethylamin und 0,53 g Bis-(trichlormethyl)-carbonat ("Triphosgen") versetzt. Anschließend wurde das Reaktionsgemisch unter Eiskühlung zu einer Lösung von 1,5 g des oben unter F) erhaltenen Diazepans in Dichlormethan zugetropft und 3 Stunden lang bei Raumtemperatur gerührt. Das Gemisch wurde dann mit 10%iger wäßriger Zitronensäurelösung geschüttelt, anschließend die organische Phase abgetrennt, über Natriumsulfat getrocknet, filtriert und im Vakuum konzentriert. Chromatographie an Kieselgel (Laufmittel: THF/MeOH) lieferte 2,88 g 1-tert.-Butyloxycarbonyl-4-{[3-(N-(3-phenylpropyl)-N-benzyloxycarbonyl]amino]propyl]aminocarbonyl}-7-phenyl-1,4-diazepan als öliges Rohprodukt, welches ohne weitere Reinigung umgesetzt wurde.
H) Es wurden 3,8 g der oben erhaltenen Diazepan-Verbindung in 50 ml Acetonitril gelöst, mit 2,5 g p-Toluolsulfonsäure versetzt und 18 Stunden lang bei Raumtemperatur gerührt. Dann wurde das Reaktionsgemisch im Vakuum eingeengt und der Rückstand über Kieselgel chromatographiert (Laufmittel: Dichlormethan/Methanol 90:10). Die vereinigten Fraktionen lieferten 1,83 g öliges 4-{[3-[N-(3-Phenylpropyl)-N-benzyloxycarbonyl]aminopropyl]aminocarbonyl}-7-phenyl-1,4-diazepan, welches ohne weitere Reinigung umgesetzt wurde.
I) Von der oben erhaltenen 1-N entschützten Diazepanverbindung wurden 1,8 g in Dichlormethan gelöst und mit 0,35 g Triethylamin und 1,0 g 3,5-Bistrifluormethyl)benzoylchlorid nach der oben in Beispiel 1 H) beschriebenen Methode umgesetzt. Nach zweimaliger Filtration über Kieselgel unter erhöhtem Druck (Laufmittel 1. Filtration: Dichlormethan, Laufmittel 2. Filtration: Dichlormethan/MeOH 98:2) erhielt man 1,66 g amorphes 1-(3,5-Bis-trifluormethylbenzoyl)-4-{[3-[N-(3-phenylpropyl)-N-benzyloxycarbonyl]aminopropyl]aminocarbonyl}-7-phenyl-1,4-diazepan, IR: 3010, 1680, 1630 cm⁻¹; M⁺: 755.
J) 1,66 g des oben erhaltenen Kopplungsproduktes wurden in 100 ml Ethanol gelöst, mit 0,5. g 10%igem Palladiumkatalysator auf Aktivkohle versetzt und bis zur Beendigung der Wasserstoffaufnahme hydriert. Anschließend filtrierte man vom Katalysator ab und engte das Filtrat im Vakuum ein. Der Rückstand wurde über Kieselgel chromatographiert (Laufmittel: THF/MeOH). Man erhielt 0,61 g der Titelverbindung, IR: 3420, 2920, 1630 cm⁻¹; M⁺: 621.

### Beispiel 6:

### 1-(3,5-Dimethylbenzoyl)-4-{2-[(N-benzyl-N-methyl)aminoethyl]}aminocarbonyl-7-phenyl-1,4-diazepan

A) 3,0 g Hexahydro-7-Phenyl-1,4-diazepin-5-on (Herstellung siehe Beispiel 1C)) in 40 ml Dichlormethan wurden mit 4,0 g Triethylamin und 2,9 g 3,5-Dimethylbenzoylchlorid nach der oben in Beispiel 1F) angegebenen Methode umgesetzt. Man erhielt 5,01 g amorphes 1-(3,5-Dimethylbenzoyl)-hexahydro-7-phenyl-1,4-diazepin-5-on, welches ohne weitere Reinigung umgesetzt wurde.
B) 3,52 g der oben hergestellten Diazepinonverbindung in 50 ml Dichlormethan wurden mit 2,28 g Triethyloxoniumtetrafluoroborat versetzt und 1,5 Stunden lang bei Raumtemperatur gerührt. Anschließend wurde die Lösung im Vakuum eingeengt und der Rückstand in 50 ml Ethanol aufgenommen. Dann wurden portionsweise 0,9 g Natriumborhydrid zugegeben und das Gemisch 18 Stunden lang bei Raumtemperatur gerührt. Anschließend wurde im Vakuum eingeengt und der Rückstand mit einem Gemisch aus Dichlormethan und Wasser aufgenommen und geschüttelt. Die organische Phase wurde abgetrennt, über Natriumsulfat getrocknet, filtriert und im Vakuum eingedampft. Chromatographie an Kieselgel (Laufmittel: Toluol/Methanol) lieferte 2,3 g 1-(3,5-Dimethylbenzoyl)-7-phenyl-1,4-diazepan, welches ohne weitere Reinigung umgesetzt wurde.
C) 1,76 g des oben hergestellten reduzierten Produktes wurden in 50 ml Dichlormethan gelöst, mit 0,6 g Chlorethylisocyanat versetzt und 2 Stunden lang bei Raumtemperatur gerührt. Nach Zugabe von Toluol wurde das Gemisch im Vakuum zur Trockene eingeengt und das entstandene 1-(3,5-Dimethylbenzoyl)-4-[(2-chlorethyl)aminocarbonyl]-7-phenyl-1,4-diazepan] als Rohprodukt weiter verarbeitet.
D) 2,3 g der oben erhaltenen Chlorethylharnstoffverbindung wurden in 80 ml Acetonitril gelöst und mit 1,0 g Diisopropylethylamin und 0,7 g N-Methylbenzylamin 18 Stunden lang unter Rückfluß zum Sieden erhitzt. Anschließend wurde die Lösung im Vakuum eingeengt und mit Methyl-tert.-butylether aufgenommen. Das Gemisch wurde mit 50 ml 10%iger wäßriger Weinsäurelösung geschüttelt und die wäßrige Phase dreimal mit Dichlormethan extrahiert. Die vereinigten organischen Phasen wurden über Natriumsulfat getrocknet, filtriert und im Vakuum eingedampft. Chromatographie an Kieselgel (Laufmittel: Dichlormethan/Methanol) lieferte 0,56 g der Titelverbindung, IR: 3015, 2920, 1630 cm⁻¹; M⁺: 498.

### Beispiel 7:

### 1-(3,5-Bistrifluormethylbenzoyl)-4-[3-(N-benzyl-N-methyl)-aminopropyl]-aminocarbonyl-7-phenyl-1,4-diazepan

A) 12,2 g N-Methylbenzylamin wurden in 100 ml Dichlormethan gelöst, mit 6,4 g Acrylnitril versetzt und 10 Minuten lang bei Raumtemperatur gerührt. Als Katalysator wurden 5 Tropfen einer 40%igen Lösung von Benzyltrimethylammoniumhydroxyd in Methanol hinzugegeben und das Gemisch anschließend 6 Stunden lang bei Raumtemperatur gerührt. Dann wurde die Lösung einmal mit 100 ml verdünnter wäßriger Essigsäure extrahiert und die organische Phase abgetrennt und über Natriumsulfat getrocknet. Es wurde vom Trockenmittel abfiltriert und im Vakuum eingeengt. Anschließend wurde der Rückstand über Kieselgel chromatographiert (Laufmittel: anfangs n-Hexan/Dichlormethan, welches mit steigenden Anteilen von Dichlormethan bis zu 100 % versetzt wurde). Man erhielt 10,4 g 3-(N-Methyl-N-benzyl)amino-propionitril, welches ohne weitere Reinigung und Charakterisierung umgesetzt wurde.
B) Das oben erhaltene Propionitril wurde in 200 ml Methanol gelöst, nacheinander mit 50 ml konzentrierter wäßriger Ammoniaklösung und 200 mg Raney-Nickel versetzt und anschließend bei Raumtemperatur und 2 bar Druck hydriert. Nach Beendigung der Wasserstoffaufnahme wurde vom Katalysator abfiltriert und das Filtrat im Vakuum eingeengt. Das erhaltene 1-[(N-Methyl-N-benzyl)amino]-1,3-diaminopropan wurde ohne weitere Reinigung für die nächste Synthesestufe eingesetzt.
C) 17,2 g Hexahydro-7-phenyl-1,4-diazepin-5-on (Herstellung siehe Beispiel 1C) in 300 ml Dichlormethan wurden mit 15,0 g Triethylamin und 25,0 g 3,5-Bistrifluormethylbenzoylchlorid nach der oben in Beispiel 1F) angegebenen Methode umgesetzt. Man erhielt 36,6 g 1-(3,5-Bistrifluormethyl)-hexahydro-7-phenyl-1,4-diazepin-5-on, Fp. = 169 - 171 °C.
D) 2,1 g der oben hergestellten Diazepinonverbindung, 1,2 g Triethyloxoniumtetrafluoroborat und 0,5 g Natriumborhydrid wurden nach der oben in Beispiel 6B) angegebenen Weise umgesetzt. Man erhielt 1,3 g 1-(3,5-Bistrifluormethylbenzoyl)-7-phenyl-1,4-diazepan vom Fp. = 151 - 153 °C.
E) 0,36 g des vorstehend unter B) erhaltenen Diaminopropans wurden in 20 ml Dichlormethan gelöst und nacheinander mit 0,3 ml Triethylamin und mit 1 ml einer 20%igen Lösung von Phosgen in Toluol versetzt. Das Gemisch wurde 2 Stunden lang bei Raumtemperatur gerührt und dann im Vakuum eingeengt. Den Rückstand löste man in 20 ml Dichlormethan und versetzte diese Lösung bei Raumtemperatur tropfenweise mit einer Lösung von 0,6 g des oben erhaltenen 1-(3,5-Bis-trifluormethylbenzoyl)-7-phenyl-1,4-diazepans und 1 ml Triethylamin in 20 ml Dichlormethan. Anschließend rührte man 2 Stunden lang bei Raumtemperatur und engte im Vakuum ein. Chromatographie an Kieselgel (Laufmittel: n-Hexan/Dichlormethan) lieferte 0,11 g der Titelverbindung; IR: 3300, 1630, 1245 cm⁻¹ (KBr); M⁺: 498.

### Beispiel 8:

### 1-(3,5-Bistrifluormethylbenzoyl)-4-{2-[N-(2-methoxybenzyl)]-aminoethyl}-7-phenyl-1,4-diazepan

A) 10,0 g N-(tert.-Butyloxycarbonyl)-glycin in 100 ml Dichlormethan wurden mit 6,6 g Triethylamin versetzt. Anschließend wurden bei 0 °C 6,2 g Chlorameisensäureethylester in 20 ml Dichlormethan zugetropft, das Gemisch 15 Minuten lang bei 0 °C gerührt und dann tropfenweise mit einer Lösung von 8,1 g 2-Methoxybenzylamin in 25 ml Dichlormethan versetzt. Man ließ das Gemisch noch 3 Stunden lang bei Raumtemperatur rühren, bevor einmal mit 100 ml einer 10%igen wäßrigen Weinsäurelösung extrahiert wurde. Die organische Phase wurde über Magnesiumsulfat getrocknet, filtriert und im Vakuum eingengt. Man erhielt 13,9 g N-(tert.-Butyloxycarbonyl)-glycin-(2-methoxybenzyl)amin vom Fp. = 96 - 97 °C.
B) 2,5 g LiAlH₄ wurden unter Stickstoffatmosphäre in einem Gemisch aus jeweils 100 ml THF und Toluol suspendiert. Hierzu wurden bei Raumtemperatur tropfenweise 13,9 g des vorstehend erhaltenen Glycinderivates, gelöst in 50 ml THF, langsam zugetropft und 4 Stunden lang bei Raumtemperatur gerührt. Unter Eiskühlung wurden dann 10 ml Wasser in 150 ml THF und anschließend 40 ml einer 5%igen wäßrigen Natronlauge hinzugetropft. Der ausgefallene Niederschlag wurde abfiltriert und das Filtrat im Vakuum eingeengt. Man erhielt 8,3 g 1-[N-(tert.-Butyloxycarbonyl)]-2-[N-(2-methoxybenzyl)amino]-1,2-diaminoethan, welches ohne weitere Reinigung für die nächste Synthesestufe eingesetzt wurde.
C) 8,3 g des vorstehend erhaltenen Diaminoethans wurden in 100 ml THF gelöst. Dazu wurden unter Eiskühlung eine Lösung von 4,0 g Chlorameisensäurebenzylester in 15 ml THF und eine Lösung von 1,0 g Natriumhydroxyd in 50 ml Wasser über 2 Tropftrichter so zugetropft, daß die Temperatur 10 °C nicht überschritt und der pH-Wert der Lösung zwischen 9,5 und 10 lag. Nach vollendeter Zugabe wurde das Reaktionsgemisch 2 Stunden lang bei Raumtemperatur gerührt. Nach Zugabe von 10 g Natriumchlorid wurde die organische Phase abgetrennt, über Magnesiumsulfat getrocknet, filtriert und im Vakuum eingeengt. Der Rückstand wurde in Dichlormethan aufgenommen und einmal mit 10%iger wäßriger Weinsäurelösung extrahiert. Die organische Phase wurde erneut abgetrennt, über Magnesiumsulfat getrocknet, filtriert und im Vakuum eingeengt. Man erhielt 5,1 g rohes 1-[N-(tert.-Butyloxycarbonyl)-2-[N-benzyloxycarbonyl-N-(2-methoxybenzyl)]amino-1,2-diaminoethan, welches ohne weitere Reinigung umgesetzt wurde.
D) 5,0 g des vorstehend erhaltenen Produktes wurden in 100 ml Acetonitril gelöst und mit 4,7 g p-Toluolsulfonsäure versetzt. Dann wurde 6 Stunden lang bei Raumtemperatur gerührt, anschließend im Vakuum eingeengt, der Rückstand in 50 ml Dichlormethan aufgenommen und einmal mit 50 ml Wasser extrahiert. Die organische Phase wurde abgetrennt, über Natriumsulfat getrocknet und bei vermindertem Druck eingeengt. Chromatographie an Kieselgel (Laufmittel: Dichlormethan/Methanol) lieferte 1,9 g 1-[N-Benzyloxycarbonyl-N-(2-methoxybenzyl)]amino-1,2-diaminoethan; IR = 3060, 3030, 2960, 1700 cm⁻¹ (KBr).
E) Man löste 0,31 g des vorstehend erhaltenen Diaminoethans in 20 ml Dichlormethan und versetzte diese Vorlage nacheinander bei 0 °C mit 0,26 g Triethylamin und 0,6 ml einer 20%igen Lösung von Phosgen in Toluol. Dann wurde das Gemisch 2 Stunden lang bei Raumtemperatur gerührt und anschließend im Vakuum eingeengt. Das entstandene {2-[N-Benzyloxycarbonyl-N-(2-methoxybenzyl)]amino}ethyl-isocyanat wurde in 10 ml Dichlormethan aufgenommen und ohne weitere Reinigung für die nächste Synthesestufe eingesetzt.
F) 0,41 g 1-(3,5-Bistrifluormethylbenzoyl)-7-phenyl-1,4-diazepan (Herstellung siehe Beispiel 7 D)) in 20 ml Dichlormethan wurden bei 0 °C tropfenweise mit der vorstehend erhaltenen Isocyanatlösung versetzt und anschließend 3 Stunden lang bei Raumtemperatur gerührt. Dann wurde einmal mit 20 ml Wasser extrahiert, die organische Phase abgetrennt, über Natriumsulfat getrocknet, filtriert und im Vakuum eingeengt. Chromatographie an Kieselgel (Laufmittel: Dichlormethan/Methanol) ergab 0,43 g 1-(3,5-Bistrifluormethylbenzoyl)-4-{2-[N-(2-methoxy-benzyl)-N-benzyloxycarbonyl)amino}ethylaminocarbonyl-7-phenyl-1,4-diazepan, IR: 3010, 1680, 1630 cm⁻¹; M⁺: 756.
G) Das vortehend erhaltene Produkt wurde in 50 ml Ethanol gelöst und mit einer Spatelspitze 10%igen Palladiumkatalysators auf Aktivkohle versetzt. Anschließend wurde bei Raumtemperatur und 3 bar Druck hydriert. Nach 3 Stunden wurde vom Katalysator abfiltriert, das Filtrat im Vakuum eingeengt und der Rückstand an Kieselgel (Laufmittel: Dichlormethan/Methanol) chromatographiert. Man erhielt 0,12 g der Titelverbindung als Öl, IR: 2420, 2920, 1630 cm⁻¹; M⁺: 622

Nach den in vorstehenden Beispielen beschriebenen Verfahren können auch die in der nachfolgenden Tabelle A angegebenen Verbindungen der Formel I hergestellt werden:

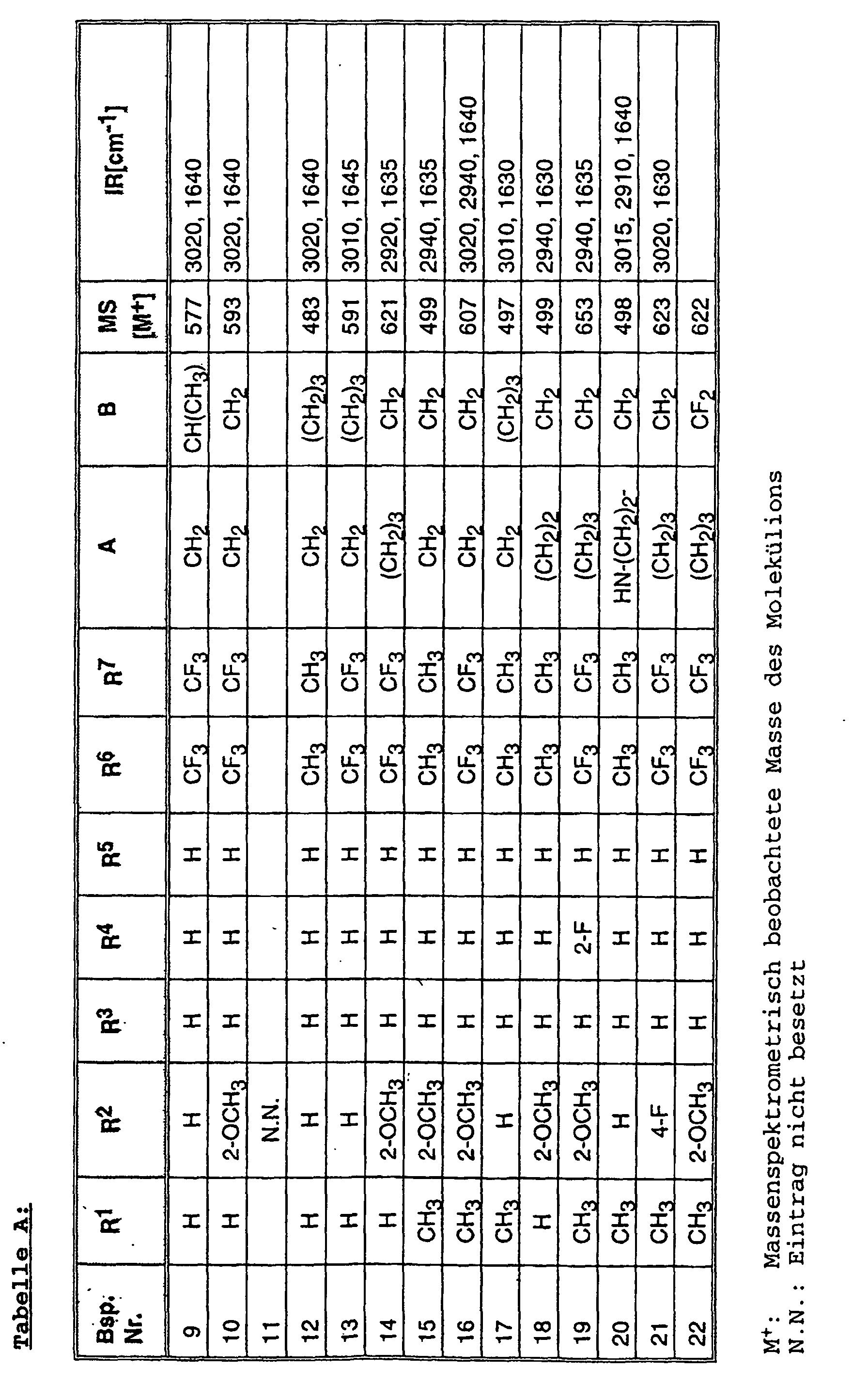

### Beispiel I:

### 1-(3,5-Bistrifluormethylbenzoyl)-4-{[3-[N-(2-methoxybenzyl)-N-methyl]amino]propylcarbonyl}-7-phenyl-1,4-diazepan enthaltende Tabletten

Man stellte Tabletten in folgender Zusammensetzung pro Tablette her:

| | |
|---|---|
| 1-(3,5-Bistrifluormethylbenzoyl)-4-{[3-[N-(2-methoxybenzyl)-N-methyl] amino]propylcarbonyl}-7-phenyl-1,4-diazepan-Hydrochlorid | 20 mg |
| Maisstärke | 60 mg |
| Milchzucker | 135 mg |
| Gelatine (als 10%ige Lösung) | 6 mg |

Der Wirkstoff, die Maisstärke und der Milchzucker wurden mit der 10%igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

## Patentansprüche

1. Verbindungen der allgemeinen Formel I, 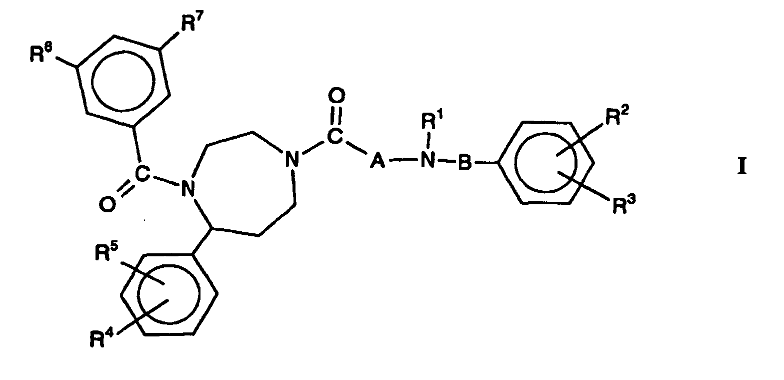 worin
R¹ Wasserstoff oder C₁₋₄-Alkyl bedeutet,
R² Wasserstoff, C₁₋₄- Alkyl, C₁₋₄-Alkoxy, Halogen oder Trifluormethyl bedeutet und
R³ Wasserstoff, C₁₋₄-Alkyl, C₁₋₄-Alkoxy, Halogen oder Trifluormethyl bedeutet, oder
R² und R³ gemeinsam an benachbarte Kohlenstoffatome des Phenylringes gebundenes Alkylendioxy mit 1 bis 2 Kohlenstoffatomen bedeuten,
R⁴ Wasserstoff, C₁₋₄-Alkyl, C₁₋₄-Alkoxy, Halogen oder Trifluormethyl bedeutet und
R⁵ Wasserstoff, C₁₋₄-Alkyl, C₁₋₄-Alkoxy, Halogen oder Trifluormethyl bedeutet, oder
R⁴ und R⁵ gemeinsam an benachbarte Kohlenstoffatome des Phenylringes gebundenes Alkylendioxy mit 1 bis 2 Kohlenstoffatomen bedeuten,
R⁶ C₁₋₄-Alkyl, Halogen oder Trifluormethyl bedeutet,
R⁷ C₁₋₄-Alkyl, Halogen oder Trifluormethyl bedeutet,
A eine -(CH₂)ₙ-Gruppe, worin n für eine ganze Zahl von 1 bis 3 steht, oder eine -NH-(CH₂)ₘ-Gruppe, worin m für eine ganze Zahl von 2 bis 3 steht, bedeutet, und
B eine gegebenenfalls durch C₁₋₄-Alkyl substituierte Alkylenkette mit 1 bis 3 Kohlenstoffatomen bedeutet,
sowie deren physiologisch verträgliche Säureadditionssalze.

2. Verbindungen gemäß Anspruch 1, worin R⁶ und/oder R⁷ Trifluormethyl bedeuten.

3. Verbindungen gemäß einem der vorstehenden Ansprüche, worin R² Wasserstoff bedeutet und R³ für 2-Methoxy steht.

4. 1-(3,5-Bistrifluormethylbenzoyl)-4-{3-[N-(2-methoxybenzyl)-N-methylamino]-propylcarbonyl}-7-phenyl-1,4-diazepan gemäß Anspruch 3 und dessen physiologisch verträgliche Säureadditionssalze.

5. Arzneimittel, enthaltend eine pharmakologisch wirksame Menge einer Verbindung gemäß Anspruch 1 und übliche pharmazeutische Hilfs- und/oder Trägerstoffe.

6. Verfahren zur Herstellung von Verbindungen der allgemeinen Formel I, 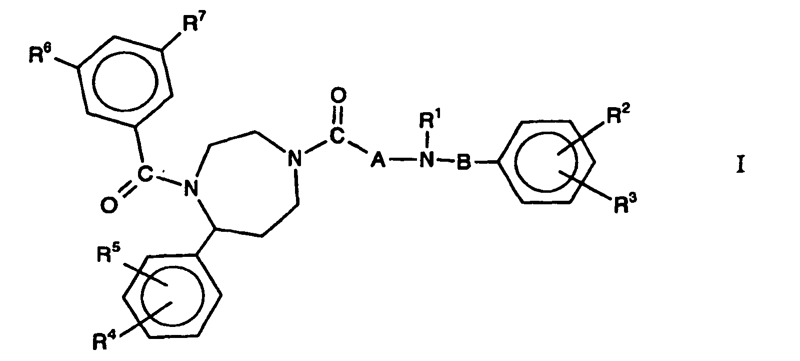 worin
R¹ Wasserstoff oder C₁₋₄-Alkyl bedeutet,
R² Wasserstoff, C₁₋₄-Alkyl, C₁₋₄-Alkoxy, Halogen oder Trifluormethyl bedeutet und
R³ Wasserstoff, C₁₋₄-Alkyl, C₁₋₄-Alkoxy, Halogen oder Trifluormethyl bedeutet, oder
R² und R³ gemeinsam an benachbarte Kohlenstoffatome des Phenylringes gebundenes Alkylendioxy mit 1 bis 2 Kohlenstoffatomen bedeuten,
R⁴ Wasserstoff, C₁₋₄-Alkyl, C₁₋₄-Alkoxy, Halogen oder Trifluormethyl bedeutet und
R⁵ Wasserstoff, C₁₋₄-Alkyl, C₁₋₄-Alkoxy, Halogen oder Trifluormethyl bedeutet, oder
R⁴ und R⁵ gemeinsam an benachbarte Kohlenstoffatome des Phenylringes gebundenes Alkylendioxy mit 1 bis 2 Kohlenstoffatomen bedeuten,
R⁶ C₁₋₄-Alkyl, Halogen oder Trifluormethyl bedeutet,
R⁷ C₁₋₄-Alkyl, Halogen oder Trifluormethyl bedeutet,
A eine -(CH₂)ₙ-Gruppe, worin n für eine ganze Zahl von 1 bis 3 steht, oder eine -NH-(CH₂)ₘ-Gruppe, worin m für eine ganze Zahl von 2 bis 3 steht, bedeutet, und
B eine gegebenenfalls durch C₁₋₄-Alkyl substituierte Alkylenkette mit 1 bis 3 Kohlenstoffatomen bedeutet,
sowie deren physiologisch verträglichen Säureadditionssalzen, **dadurch gekennzeichnet, daß** man
a) zur Herstellung von Verbindungen der Formel I Verbindungen der allgemeinen Formel IIa, 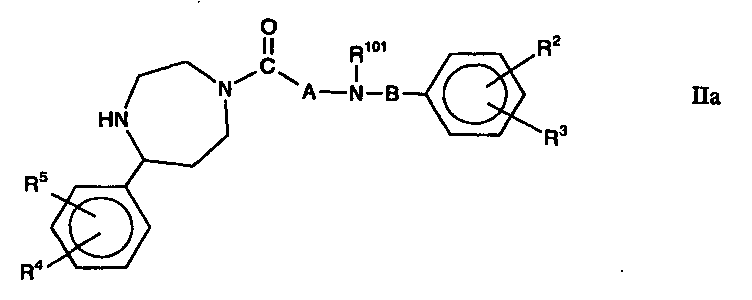 worin R², R³, R⁴, R⁵, A und B obige Bedeutungen besitzen und R¹⁰¹ für C₁₋₄-Alkyl oder eine Aminoschutzgruppe steht, mit Verbindungen der allgemeinen Formel III, 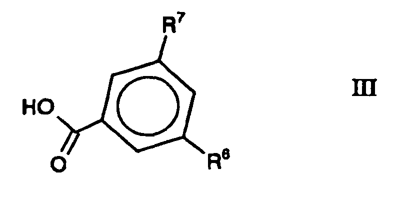 worin R⁶ und R⁷ obige Bedeutungen besitzen, umsetzt und eine allfällige Aminoschutzgruppe R¹⁰¹ wieder abspaltet, oder
b) zur Herstellung von Verbindungen der allgemeinen Formel Ia, 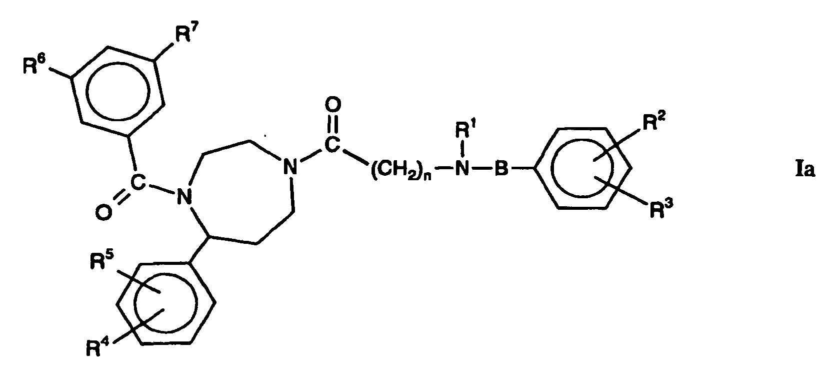 worin R¹, R², R³, R⁴, R⁵, R⁶, R⁷, B und n obige Bedeutungen besitzen, Verbindungen der allgemeinen Formel IV, 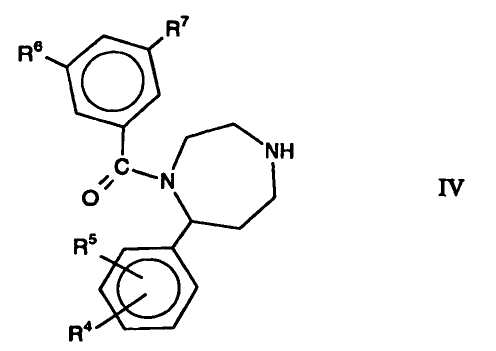 worin R⁴, R⁵, R⁶ und R⁷ obige Bedeutungen besitzen, mit Verbindungen der allgemeinen Formel V, 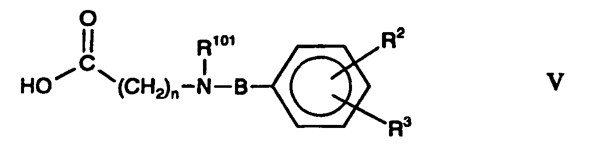 worin R¹⁰¹, R², R³, B und n obige Bedeutungen besitzen, umsetzt und eine allfällige Aminoschutzgruppe R¹⁰¹ wieder abspaltet, oder
c) zur Herstellung von Verbindungen der allgemeinen Formel Ib, 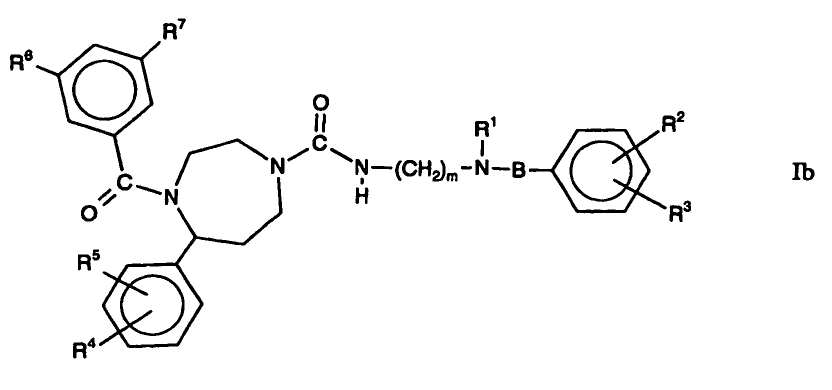 worin R¹, R², R³, R⁴, R⁵, R⁶ , R⁷, B und m obige Bedeutungen besitzen, Verbindungen der Formel IV mit Verbindungen der allgemeinen Formel VI, 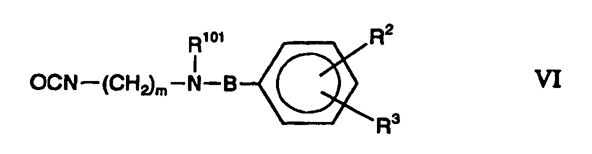 worin R¹⁰¹, R², R³, B und m obige Bedeutungen besitzen, umsetzt und eine allfällige Aminoschutzgruppe R¹⁰¹ wieder abspaltet, oder
d) zur Herstellung von Verbindungen der Formel I Verbindungen der allgemeinen Formel VIII, 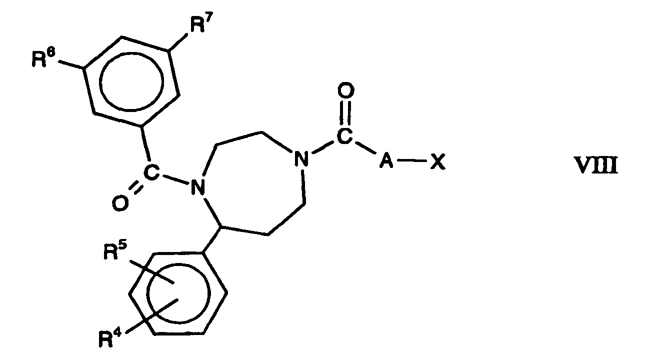 worin R⁴, R⁵, R⁶, R⁷ und A obige Bedeutungen besitzen und X für eine abspaltbare Fluchtgruppe steht, mit Verbindungen der allgemeinen Formel IXa, 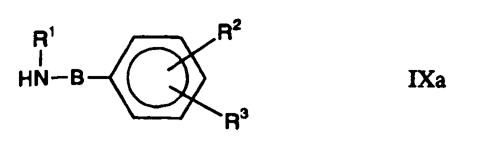 worin R¹, R², R³ und B obige Bedeutungen besitzen, umsetzt, oder
e) zur Herstellung von Verbindungen der Formel I Verbindungen der allgemeinen Formel X, 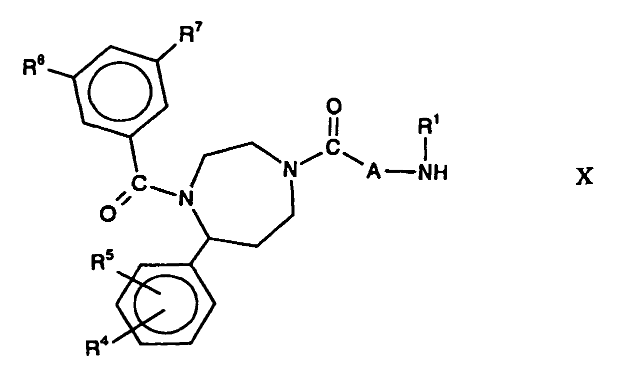 worin R¹, R⁴, R⁵, R⁶, R⁷ und A obige Bedeutungen besitzen, mit Verbindungen der allgemeinen Formel XIa, 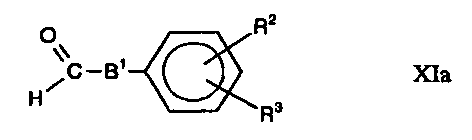 worin R² und R³ obige Bedeutungen besitzen und B¹ für eine Bindung oder eine gegebenenfalls durch C₁₋₄-Alkyl substituierte Alkylenkette mit 1 bis 2 Kohlenstoffatomen steht, unter Bedingungen der reduktiven Alkylierung umsetzt oder mit Verbindungen der allgemeinen Formel XIb, 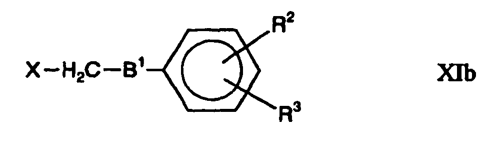 worin R², R³, B¹ und X obige Bedeutungen besitzen, alkyliert,
und gewünschtenfalls erhaltene Verbindungen der Formel I, worin R¹ Wasserstoff bedeutet, zu Verbindungen der Formel I, worin R¹ C₁₋₄-Alkyl bedeutet, alkyliert und erhaltene Verbindungen der allgemeinen Formel I gewünschtenfalls in ihre Säureadditionssalze überführt oder Säureadditionssalze in freie Verbindungen der Formel I überführt.

7. Verbindungen der allgemeinen Formel II, 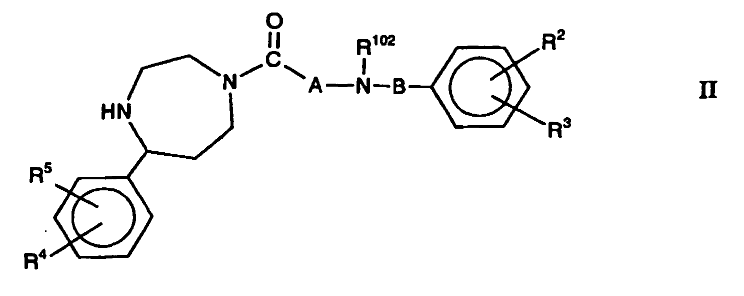 worin
R¹⁰² Wasserstoff, C₁₋₄-Alkyl oder eine Aminoschutzgruppe bedeutet,
R² Wasserstoff, C₁₋₄-Alkyl, C₁₋₄-Alkoxy, Halogen oder Trifluormethyl bedeutet und
R³ Wasserstoff, C₁₋₄-Alkyl, C₁₋₄-Alkoxy, Halogen oder Trifluormethyl bedeutet, oder
R² und R³ gemeinsam an benachbarte Kohlenstoffatome des Phenylringes gebundenes Alkylendioxy mit 1 bis 2 Kohlenstoffatomen bedeuten,
R⁴ Wasserstoff, C₁₋₄-Alkyl, C₁₋₄-Alkoxy, Halogen oder Trifluormethyl bedeutet und
R⁵ Wasserstoff, C₁₋₄-Alkyl, C₁₋₄-Alkoxy, Halogen oder Trifluormethyl bedeutet, oder
R⁴ und R⁵ gemeinsam an benachbarte Kohlenstoffatome des Phenylringes gebundenes Alkylendioxy mit 1 bis 2 Kohlenstoffatomen bedeuten,
A eine -(CH₂)ₙ-Gruppe, worin n für eine ganze Zahl von 1 bis 3 steht, oder eine -NH-(CH₂)ₘ-Gruppe, worin m für eine ganze Zahl von 2 bis 3 steht, bedeutet, und
B eine gegebenenfalls durch C₁₋₄-Alkyl substituierte Alkylenkette mit 1 bis 3 Kohlenstoffatomen bedeutet.

8. Verbindungen der allgemeinen Formel IV, 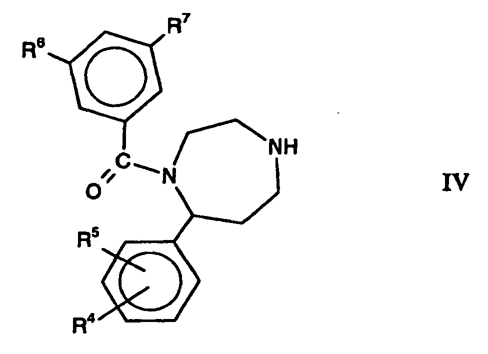 worin
R⁴ Wasserstoff, C₁₋₄-Alkyl, C₁₋₄-Alkoxy, Halogen oder Trifluormethyl bedeutet und
R⁵ Wasserstoff, C₁₋₄-Alkyl, C₁₋₄-Alkoxy, Halogen oder Trifluormethyl bedeutet, oder
R⁴ und R⁵ gemeinsam an benachbarte Kohlenstoffatome des Phenylringes gebundenes Alkylendioxy mit 1 bis 2 Kohlenstoffatomen bedeuten,
R⁶ C₁₋₄-Alkyl, Halogen oder Trifluormethyl bedeutet und
R⁷ C₁₋₄-Alkyl, Halogen oder Trifluormethyl bedeutet.

9. Verbindungen der allgemeinen Formel VIII 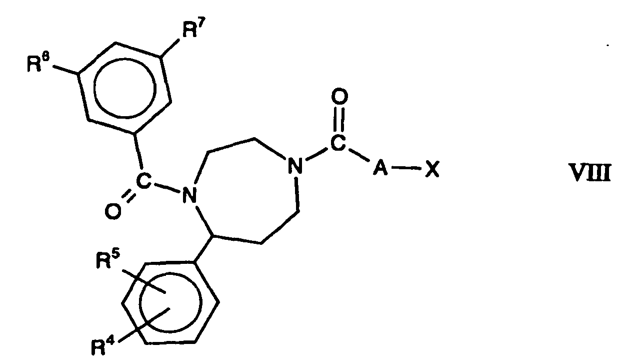 worin
R⁴ Wasserstoff, C₁₋₄-Alkyl, C₁₋₄-Alkoxy, Halogen oder Trifluormethyl bedeutet und
R⁵ Wasserstoff, C₁₋₄-Alkyl, C₁₋₄-Alkoxy, Halogen oder Trifluormethyl bedeutet, oder
R⁴ und R⁵ gemeinsam an benachbarte Kohlenstoffatome des Phenylringes gebundenes Alkylendioxy mit 1 bis 2 Kohlenstoffatomen bedeuten,
R⁶ C₁₋₄-Alkyl, Halogen oder Trifluormethyl bedeutet,
R⁷ C₁₋₄-Alkyl, Halogen oder Trifluormethyl bedeutet,
A eine -(CH₂)ₙ-Gruppe, worin n für eine ganze Zahl von 1 bis 3 steht, oder eine -NH-(CH₂)ₘ-Gruppe, worin m für eine ganze Zahl von 2 bis 3 steht, bedeutet, und
X für eine abspaltbare Fluchtgruppe steht.

10. Verbindungen der allgemeinen Formel X 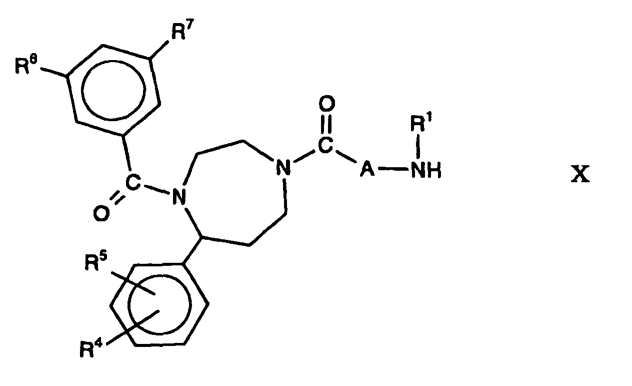 worin
R¹ Wasserstoff oder C₁₋₄-Alkyl bedeutet,
R⁴ Wasserstoff, C₁₋₄-Alkyl, C₁₋₄-Alkoxy, Halogen oder Trifluormethyl bedeutet und
R⁵ Wasserstoff, C₁₋₄-Alkyl, C₁₋₄-Alkoxy, Halogen oder Trifluormethyl bedeutet, oder
R⁴ und R⁵ gemeinsam an benachbarte Kohlenstoffatome des Phenylringes gebundenes Alkylendioxy mit 1 bis 2 Kohlenstoffatomen bedeuten,
R⁶ C₁₋₄-Alkyl, Halogen oder Trifluormethyl bedeutet,
R⁷ C₁₋₄-Alkyl, Halogen oder Trifluormethyl bedeutet, und
A eine -(CH₂)ₙ-Gruppe, worin n für eine ganze Zahl von 1 bis 3 steht, oder eine -NH-(CH₂)ₘ-Gruppe, worin m für eine ganze Zahl von 2 bis 3 steht, bedeutet.

## Claims

1. Compounds of the general formula I, 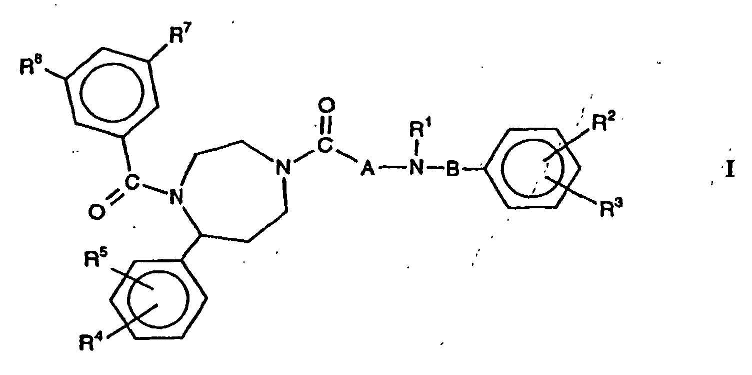 wherein
R¹ is hydrogen or C₁₋₄ alkyl,
R² is hydrogen, C₁₋₄ alkyl, C₁₋₄ alkoxy, halogen or trifluoromethyl, and
R³ is hydrogen, C₁₋₄ alkyl, C₁₋₄ alkoxy, halogen or trifluoromethyl, or
R² and R³ together are alkylenedioxy with 1 to 2 carbon atoms, bonded to adjacent carbon atoms of the phenyl ring,
R⁴ is hydrogen, C₁₋₄ alkyl, C₁₋₄ alkoxy, halogen or trifluoromethyl, and
R⁵ is hydrogen, C₁₋₄ alkyl, C₁₋₄ alkoxy, halogen or trifluoromethyl, or
R⁴ and R⁵ together are alkylenedioxy with 1 to 2 carbon atoms, bonded to adjacent carbon atoms of the phenyl ring,
R⁶ is C₁₋₄ alkyl, halogen or trifluoromethyl,
R⁷ is C₁₋₄ alkyl, halogen or trifluoromethyl,
A is a -(CH₂)ₙ- group in which n stands for an integer from 1 to 3, or an -NH-(CH₂)ₘ- group in which m stands for an integer from 2 to 3, and
B is an alkylene chain with 1 to 3 carbon atoms, optionally substituted by C₁₋₄ alkyl,
and physiologically compatible acid addition salts thereof.

2. Compounds according to Claim 1, wherein R⁶ and/or R⁷ are trifluoromethyl.

3. Compounds according to one of the preceding claims, wherein R² is hydrogen and R³ stands for 2-methoxy.

4. 1-(3,5-bistrifluoromethylbenzoyl)-4-{3-[N-(2-methoxybenzyl)-N-methylamino]-propylcarbonyl}-7-phenyl-1,4-diazepane according to Claim 3 and the physiologically compatible acid addition salts thereof.

5. Medicament, containing a pharmacologically effective quantity of a compound according to Claim 1 and conventional pharmaceutical auxiliaries and/or excipients.

6. A process for the preparation of compounds of the general formula I, 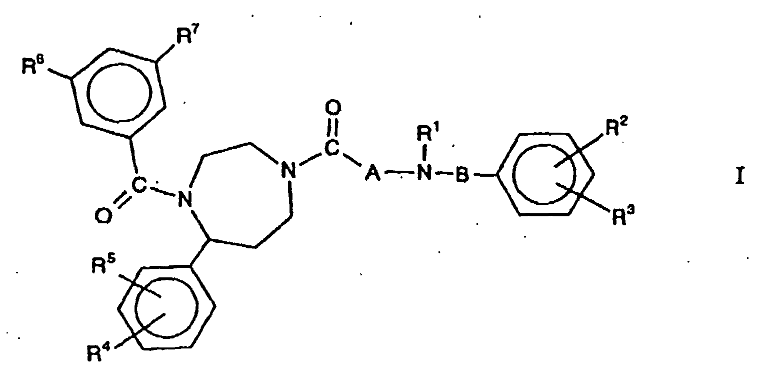 wherein
R¹ is hydrogen or C₁₋₄ alkyl,
R² is hydrogen, C₁₋₄ alkyl, C₁₋₄ alkoxy, halogen or trifluoromethyl, and
R³ is hydrogen, C₁₋₄ alkyl, C₁₋₄ alkoxy, halogen or trifluoromethyl, or
R² and R³ together are alkylenedioxy with 1 to 2 carbon atoms, bonded to adjacent carbon atoms of the phenyl ring,
R⁴ is hydrogen, C₁₋₄ alkyl, C₁₋₄ alkoxy, halogen or trifluoromethyl, and
R⁵ is hydrogen, C₁₋₄ alkyl, C₁₋₄ alkoxy, halogen or trifluoromethyl, or
R⁴ and R⁵ together are alkylenedioxy with 1 to 2 carbon atoms, bonded to adjacent carbon atoms of the phenyl ring,
R⁶ is C₁₋₄ alkyl, halogen or trifluoromethyl,
R⁷ is C₁₋₄ alkyl, halogen or trifluoromethyl,
A is a -(CH₂)ₙ- group in which n stands for an integer from 1 to 3, or an -NH-(CH₂)ₘ- group in which m stands for an integer from 2 to 3, and
B is an alkylene chain with 1 to 3 carbon atoms, optionally substituted by C₁₋₄ alkyl,
and physiologically compatible acid addition salts thereof,
**characterised in that**
a) for the preparation of compounds of Formula I compounds of the general formula IIa 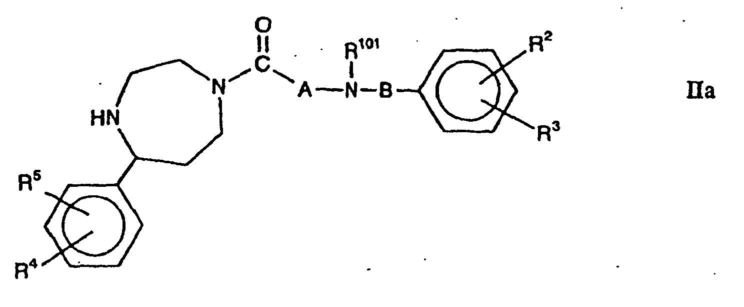 wherein R², R³, R⁴, R⁵, A and B have the above meanings and R¹⁰¹ stands for C₁₋₄ alkyl or an amino protective group, are reacted with compounds of the general formula III, 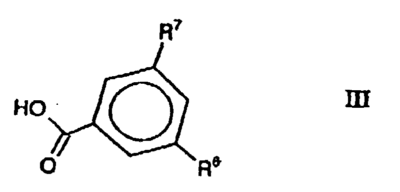 wherein R⁶ and R⁷ have the above meanings, and any amino protective group R¹⁰¹ is cleaved off again, or
b) for the preparation of compounds of the general formula Ia, 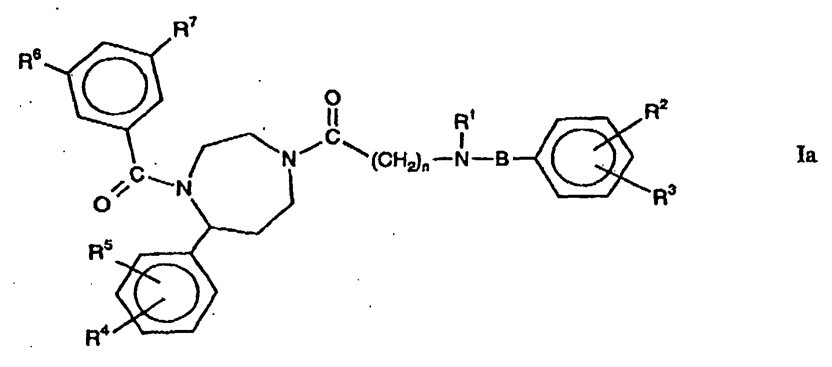 wherein R¹, R², R³, R⁴, R⁵, R⁶, R⁷, B and n have the above meanings, compounds of the general formula IV, 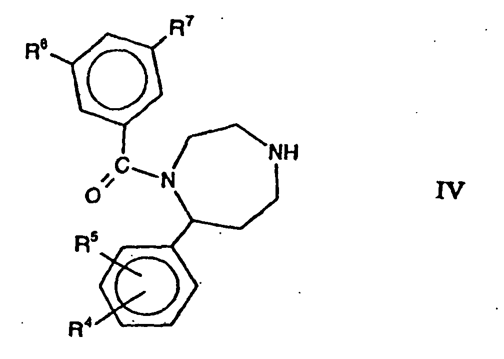 wherein R⁴, R⁵, R⁶ and R⁷ have the above meanings, are reacted with compounds of the general formula V, 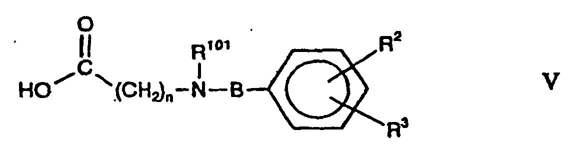 wherein R¹⁰¹, R², R³, B and n have the above meanings, and any amino protective group R¹⁰¹ is cleaved off again, or
c) for the preparation of compounds of the general formula Ib, 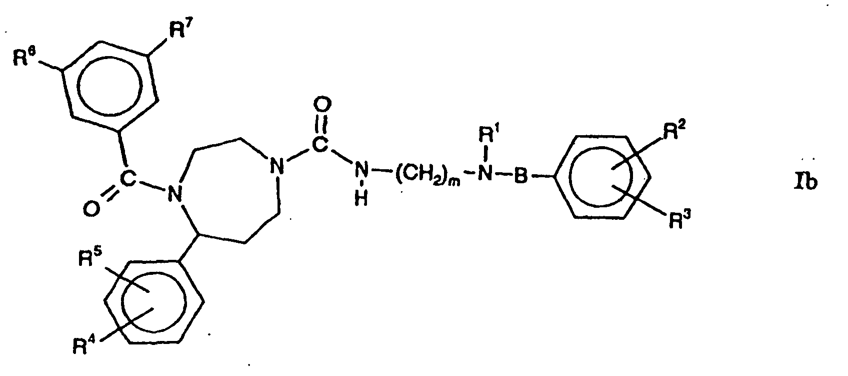 wherein R¹, R², R³, R⁴, R⁵, R⁶ , R⁷, B and m have the above meanings, compounds of Formula IV are reacted with compounds of the general formula VI, 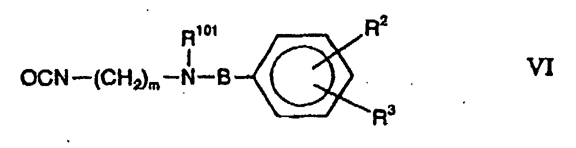 wherein R¹⁰¹, R², R³, B and m have the above meanings, and any amino protective group R¹⁰¹ is cleaved off again, or
d) for the preparation of compounds of Formula I, compounds of the general formula VIII, 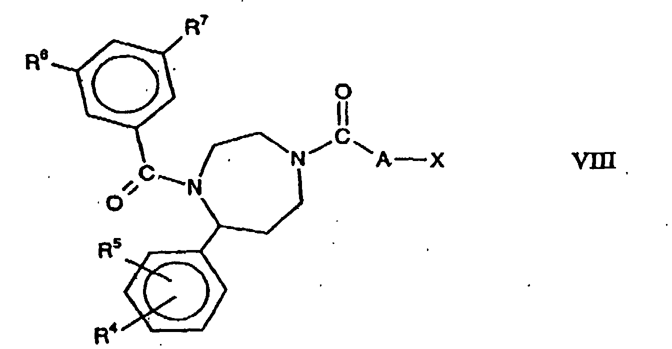 wherein R⁴, R⁵, R⁶, R⁷ and A have the above meanings and X stands for a cleavable leaving group, are reacted with compounds of the general formula IXa, 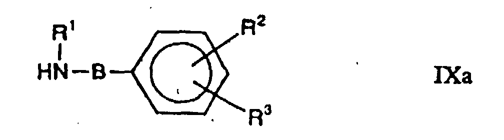 wherein R¹, R², R³ and B have the above meanings, or
e) for the preparation of compounds of Formula I, compounds of the general formula X, 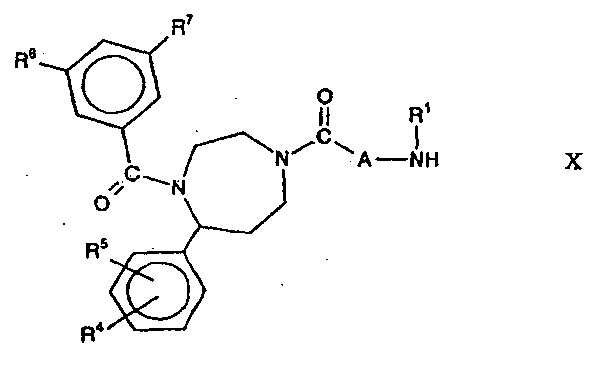 wherein R¹, R⁴, R⁵, R⁶, R⁷ and A have the above meanings, are reacted under conditions of reductive alkylation with compounds of the general formula XIa, 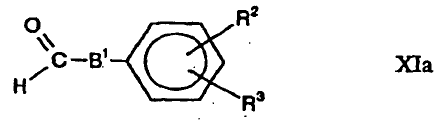 wherein R² and R³ have the above meanings and B¹ stands for a bond or an alkylene chain with 1 to 2 carbon atoms, optionally substituted by C₁₋₄ alkyl, or are alkylated with compounds of the general formula XIb, 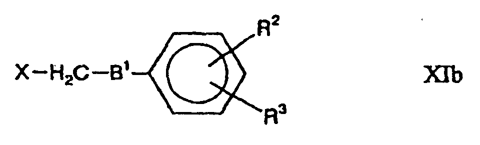 wherein R², R³, B¹ and X have the above meanings,
and if desired resulting compounds of Formula I wherein R¹ is hydrogen are alkylated to form compounds of Formula I wherein R¹ is C₁₋₄ alkyl, and resulting compounds of the general formula I if desired are converted into the acid addition salts thereof or acid addition salts are converted into free compounds of Formula I.

7. Compounds of the general formula II, 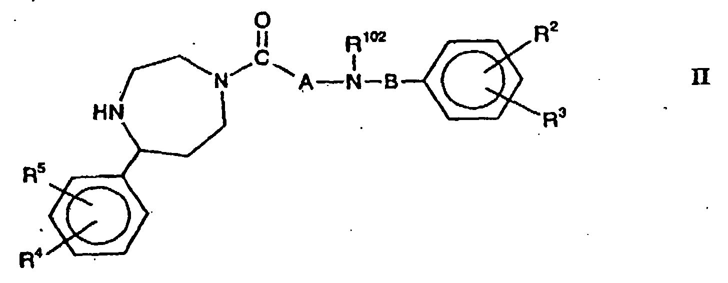 wherein
R¹⁰² is hydrogen, C₁₋₄ alkyl or an amino protective group,
R² is hydrogen, C₁₋₄ alkyl, C₁₋₄ alkoxy, halogen or trifluoromethyl, and
R³ is hydrogen, C₁₋₄ alkyl, C₁₋₄ alkoxy, halogen or trifluoromethyl, or
R² and R³ xtogether are alkylenedioxy with 1 to 2 carbon atoms, bonded to adjacent carbon atoms of the phenyl ring,
R⁴ is hydrogen, C₁₋₄ alkyl, C₁₋₄ alkoxy, halogen or trifluoromethyl, and
R⁵ is hydrogen, C₁₋₄ alkyl, C₁₋₄ alkoxy, halogen or trifluoromethyl, or
R⁴ and R⁵ together are alkylenedioxy with 1 to 2 carbon atoms, bonded to adjacent carbon atoms of the phenyl ring,
A is a -(CH₂)ₙ- group in which n stands for an integer from 1 to 3, or an -NH-(CH₂)ₘ- group in which m stands for an integer from 2 to 3, and
B is an alkylene chain with 1 to 3 carbon atoms, optionally substituted by C₁₋₄ alkyl.

8. Compounds of the general formula IV, 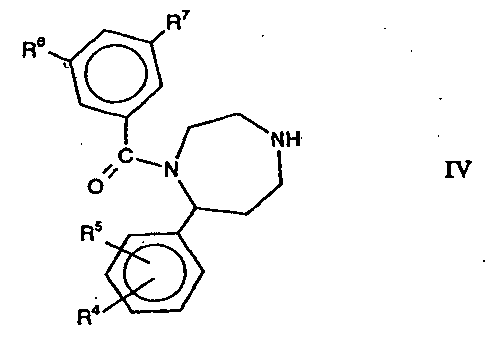 wherein
R⁴ is hydrogen, C₁₋₄ alkyl, C₁₋₄ alkoxy, halogen or trifluoromethyl, and
R⁵ is hydrogen, C₁₋₄ alkyl, C₁₋₄ alkoxy, halogen or trifluoromethyl, or
R⁴ and R⁵ together are alkylenedioxy with 1 to 2 carbon atoms, bonded to adjacent carbon atoms of the phenyl ring,
R⁶ is C₁₋₄ alkyl, halogen or trifluoromethyl, and
R⁷ is C₁₋₄ alkyl, halogen or trifluoromethyl.

9. Compounds of the general formula VIII, 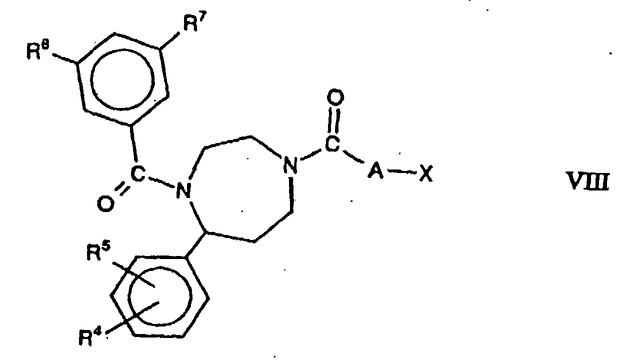 wherein
R⁴ is hydrogen, C₁₋₄ alkyl, C₁₋₄ alkoxy, halogen or trifluoromethyl, and
R⁵ is hydrogen, C₁₋₄ alkyl, C₁₋₄ alkoxy, halogen or trifluoromethyl, or
R⁴ and R⁵ together are alkylenedioxy with 1 to 2 carbon atoms, bonded to adjacent carbon atoms of the phenyl ring,
R⁶ is C₁₋₄ alkyl, halogen or trifluoromethyl,
R⁷ is C₁₋₄ alkyl, halogen or trifluoromethyl,
A is a -(CH₂)ₙ- group in which n stands for an integer from 1 to 3, or an -NH-(CH₂)ₘ- group in which m stands for an integer from 2 to 3, and
X stands for a cleavable leaving group.

10. Compounds of the general formula X, 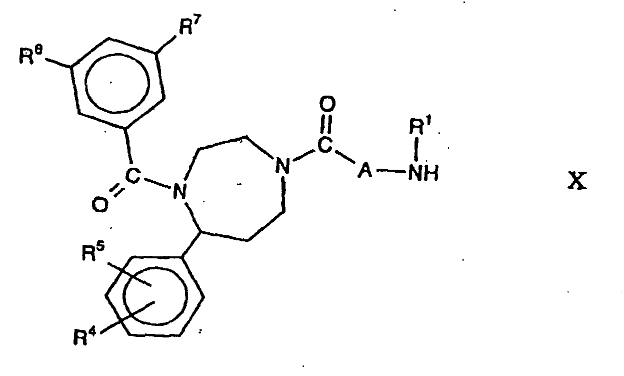 wherein
R¹ is hydrogen or C₁₋₄ alkyl,
R⁴ is hydrogen, C₁₋₄ alkyl, C₁₋₄ alkoxy, halogen or trifluoromethyl, and
R⁵ is hydrogen, C₁₋₄ alkyl, C₁₋₄ alkoxy, halogen or trifluoromethyl, or
R⁴ and R⁵ together are alkylenedioxy with 1 to 2 carbon atoms, bonded to adjacent carbon atoms of the phenyl ring,
R⁶ is C₁₋₄ alkyl, halogen or trifluoromethyl,
R⁷ is C₁₋₄ alkyl, halogen or trifluoromethyl, and
A is a -(CH₂)ₙ- group in which n stands for an integer from 1 to 3, or an -NH-(CH₂)ₘ- group in which m stands for an integer from 2 to 3.

## Revendications

1. Composés de formule générale I 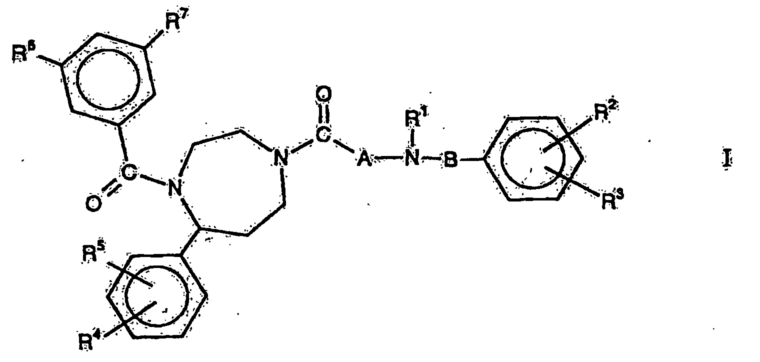 dans laquelle
R¹ représente un hydrogène ou un alkyle en C₁ à C₄,
R² représente un hydrogène, alkyle en C₁ à C₄, alcoxy en C₁ à C₄, halogène ou trifluorométhyle, et
R³ représente un hydrogène, alkyle en C₁ à C₄, alcoxy en C₁ à C₄, halogène ou trifluorométhyle, ou
R² et R³ représentent ensemble un alkylènedioxy comportant 1 ou 2 atomes de carbone lié à des atomes de carbone voisins du noyau phényle,
R⁴ représente un hydrogène, alkyle en C₁ à C₄, alcoxy en C₁ à C₄, halogène ou trifluorométhyle, et
R⁵ représente un hydrogène, alkyle en C₁ à C₄, alcoxy en C₁ à C₄, halogène ou trifluorométhyle, ou
R⁴ et R⁵ représentent ensemble un alkylènedioxy ayant 1 ou 2 atomes de carbone liés à des atomes de carbone voisins du noyau phényle,
R⁶ représente un alkyle en C₁ à C₄, halogène, ou trifluorométhyle,
R⁷ représente un alkyle en C₁ à C₄, halogène ou trifluorométhyle,
A représente un groupe -(CH₂)ₙ, dans lequel n représente un nombre entier de 1 à 3, ou un groupe -NH-(CH₂)ₘ, où m représente un nombre entier de 2 ou 3, et
B représente une chaîne alkylène ayant 1 à 3 atomes de carbone éventuellement substituée par un alkyle en C₁ à C₄,
ainsi que leurs sels d'addition d'acides physiologiquement compatibles.

2. Composés selon la revendication 1, dans lesquels R⁶ et/ou R⁷ représentent un trifluorométhyle.

3. Composés selon l'une quelconque des revendications précédentes, dans lesquels R² représente l'hydrogène et R³ est un 2-méthoxy.

4. 1-(3,5-bistrifluorométhylbenzoyl)-4- {3-[N-(2-méthoxy-benzyl)-N-méthylamino]-propylcarbonyl}-7-phényl-1,4-diazépan selon la revendication 3 et ses sels d'addition d'acides physiologiquement compatibles.

5. Médicament contenant une quantité pharmacologiquement efficace d'un composé selon la revendication 1 et des additifs et/ou excipients pharmaceutiques habituels.

6. Procédé de préparation de composés de formule générale I 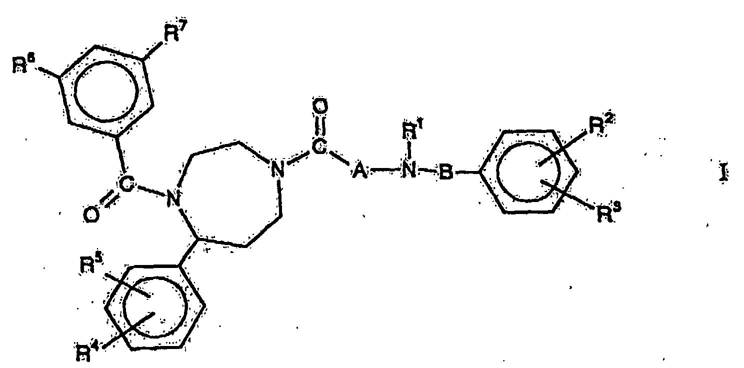 dans laquelle
R¹ représente un hydrogène ou un alkyle en C₁ à C₄,
R² représente un hydrogène, alkyle en C₁ à C₄, alcoxy en C₁ à C₄, halogène ou trifluorométhyle, et
R³ représente un hydrogène, alkyle en C₁ à C₄, alcoxy en C₁ à C₄, halogène ou trifluorométhyle, ou
R² et R³ représentent ensemble un alkylènedioxy comportant de 1 ou 2 atomes de carbone lié à des atomes de carbone voisins du noyau phényle,
R⁴ représente un hydrogène, alkyle en C₁ à C₄, alcoxy en C₁ à C₄, halogène ou trifluorométhyle, et
R⁵ représente un hydrogène, alkyle en C₁ à C₄, alcoxy en C₁ à C₄, halogène ou trifluorométhyle, ou
R⁴ et R⁵ représentent ensemble un alkylènedioxy ayant 1 ou 2 atomes de carbone lié aux atomes de carbone voisins du noyau phényle,
R⁶ représente un alkyle en C₁ à C₄, halogène, ou trifluorométhyle,
R⁷ représente un alkyle en C₁ à C₄, halogène ou trifluorométhyle,
A représente un groupe -(CH₂)ₙ, dans lequel n représente un nombre entier compris entre 1 et 3, ou un groupe -NH-(CH₂)ₘ, où m représente un nombre entier de 2 ou 3, et
B représente une chaîne alkylène ayant 1 à 3 atomes de carbone éventuellement substituée par un alkyle en C₁ à C₄, ainsi que de leurs sels d'addition d'acides physiologiquement compatibles,
**caractérisé en ce que** l'on procède
a) pour préparer des composés de formule I en faisant réagir des composés de formule générale IIa 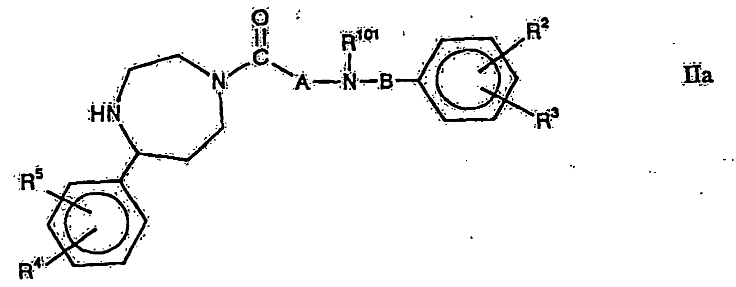 dans laquelle R², R³, R⁴, R⁵, A et B ont les significations données ci-avant et R¹⁰¹ représente un alkyle en C₁ à C₄ ou un groupe protecteur amino, avec des composés de formule générale III 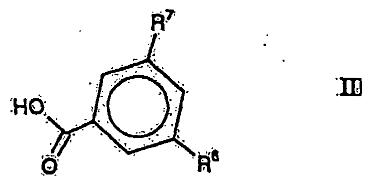 dans laquelle R⁶ et R⁷ ont les significations données ci-avant, et en séparant ensuite à nouveau un groupe protecteur amino R¹⁰¹ éventuellement présent, ou
b) pour préparer des composés de formule générale Ia 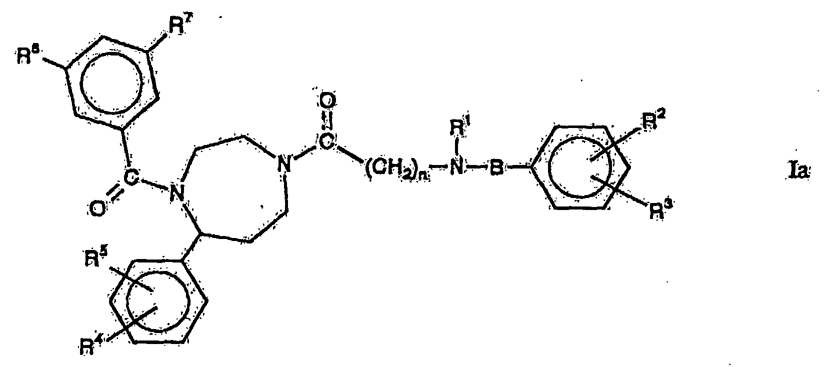 dans laquelle R¹, R², R³, R⁴, R⁵, R⁶, R⁷, B et n ont les significations données ci-avant, en faisant réagir des composés de formule générale IV, 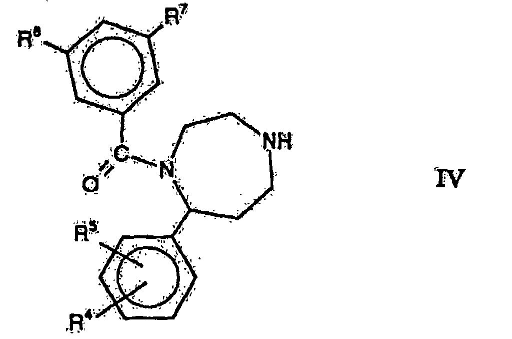 dans laquelle R⁴, R⁵, R⁶ et R⁷ ont les significations données ci-avant, avec des composés de formule générale V, 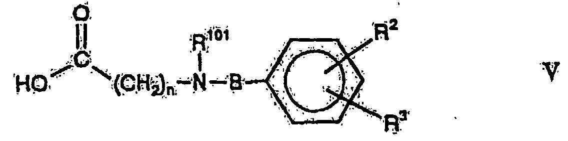 dans laquelle R¹⁰¹, R², R³, B et n ont la signification donnée ci-avant et en séparant ensuite à nouveau un groupe protecteur amino R¹⁰¹ éventuellement présent, ou
c) pour préparer des composés de formule générale Ib, 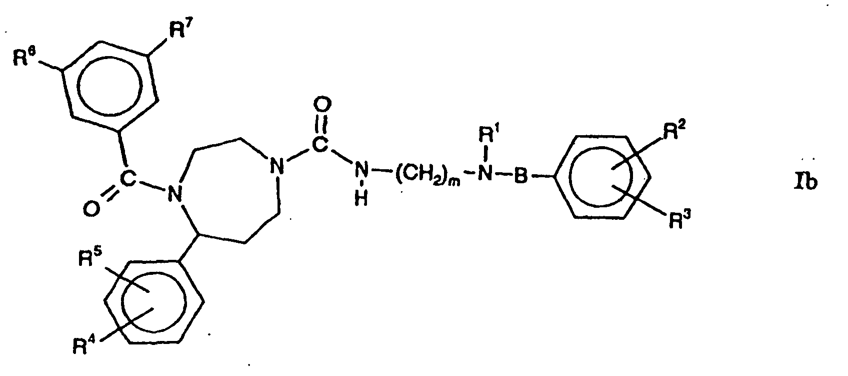 dans laquelle R¹, R², R³, R⁴, R⁵, R⁶, R⁷, B et m ont les significations données ci-avant, en faisant réagir des composés de formule IV avec des composés de formule générale VI, 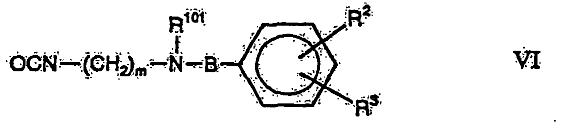 dans laquelle R¹⁰¹, R², R³, B et m ont les significations données ci-avant et en séparant ensuite à nouveau un groupe protecteur amino R¹⁰¹ éventuellement présent, ou
d) pour préparer des composés de formule I, en faisant réagir des composés de formule générale VIII, 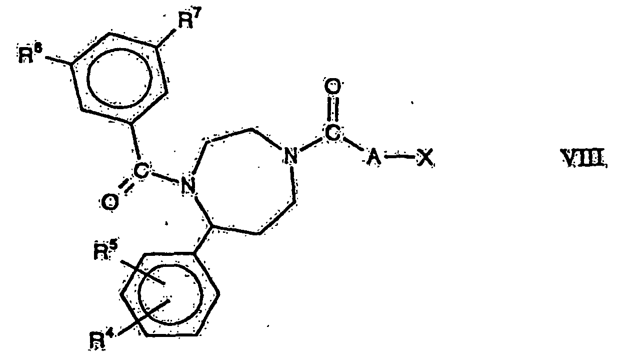 dans laquelle R⁴, R⁵, R⁶, R⁷ et A ont les signification données ci-avant et X représente un groupe partant séparable, avec des composés de formule générale IXa, 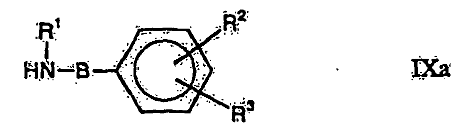 dans laquelle R¹, R², R³ et B ont les significations données ci-avant, ou
e) pour préparer des composés de formule I, en faisant réagir des composés de formule générale X, 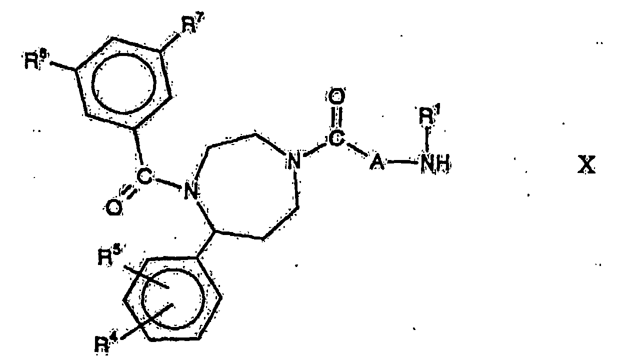 dans laquelle R¹, R⁴, R⁵, R⁶, R⁷ et A ont les significations données ci-avant, avec des composés de formule générale XIa, 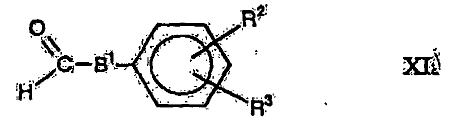 dans laquelle R² et R³ ont les significations données ci-avant et B¹ représente une liaison ou une chaîne alkylène ayant 1 ou 2 atomes de carbone éventuellement substituée par un alkyle en C₁ à C₄, dans les conditions d'une alkylation réductrice ou en alkylant avec des composés de formule générale XIb, 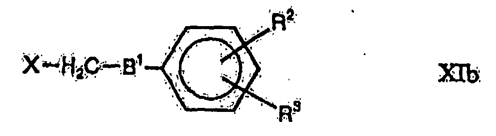 dans laquelle R², R³, B¹ et X ont les significations données ci-avant,
et si on le désire en alkylant les composés de formule I obtenus dans laquelle R¹ représente un hydrogène en composés de formule I dans laquelle R¹ représente un alkyle en C₁ à C₄, et, si on le désire, en transformant les composés de formule générale I obtenus en leurs sels d'addition d'acides ou en transformant les sels d'addition d'acides en composés libres de formule I.

7. Composés de formule générale II, 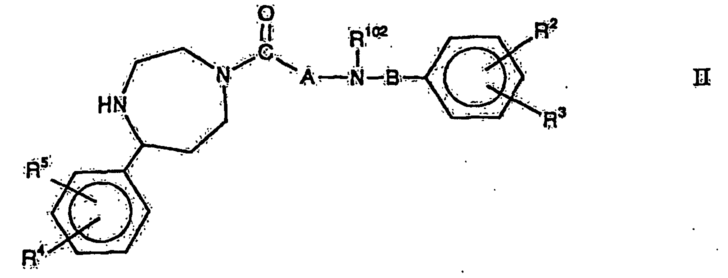 dans laquelle
R¹⁰² représente un hydrogène, un alkyle en C₁ à C₄, ou un groupe protecteur amino,
R² représente un hydrogène, alkyle en C₁ à C₄, alcoxy en C₁ à C₄, halogène ou trifluorométhyle, et
R³ représente un hydrogène, alkyle en C₁ à C₄, alcoxy en C₁ à C₄, halogène ou trifluorométhyle, ou
R² et R³ représentent ensemble un alkylènedioxy comportant 1 ou 2 atomes de carbone lié aux atomes de carbone voisins du noyau phényle,
R⁴ représente un hydrogène, alkyle en C₁ à C₄, alcoxy en C₁ à C₄, halogène ou trifluorométhyle, et
R⁵ représente un hydrogène, alkyle en C₁ à C₄, alcoxy en C₁ à C₄, halogène ou trifluorométhyle, ou
R⁴ et R⁵ représentent ensemble un alkylènedioxy ayant 1 ou 2 atomes de carbone lié aux atomes de carbone voisins du noyau phényle,
A représente un groupe -(CH₂)ₙ, dans lequel n représente un nombre entier compris entre 1 et 3, ou un groupe -NH-(CH₂)ₘ, où m représente un nombre entier de 2 ou 3,
B représente une chaîne alkylène ayant 1 à 3 atomes de carbone éventuellement substituée par un alkyle en C₁ à C₄.

8. Composés de formule générale IV, 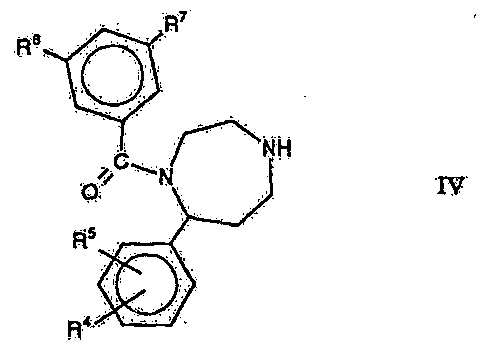 dans laquelle
R⁴ représente un hydrogène, alkyle en C₁ à C₄, alcoxy en C₁ à C₄, halogène ou trifluorométhyle, et
R⁵ représente un hydrogène, alkyle en C₁ à C₄, alcoxy en C₁ à C₄, halogène ou trifluorométhyle, ou
R⁴ et R⁵ représentent ensemble un alkylènedioxy ayant 1 ou 2 atomes de carbone lié aux atomes de carbone voisins du noyau phényle,
R⁶ représente un alkyle en C₁ à C₄, halogène, ou trifluorométhyle,
R⁷ représente un alkyle en C₁ à C₄, halogène ou trifluorométhyle,

9. Composés de formule générale VIII, 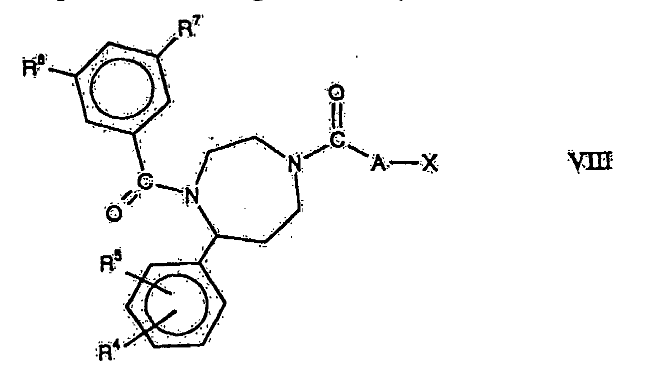 dans laquelle
R⁴ représente un hydrogène, alkyle en C₁ à C₄, alcoxy en C₁ à C₄, halogène ou trifluorométhyle, et
R⁵ représente un hydrogène, alkyle en C₁ à C₄, alcoxy en C₁ à C₄, halogène ou trifluorométhyle, ou
R⁴ et R⁵ représentent ensemble un alkylènedioxy ayant 1 ou 2 atomes de carbone lié aux atomes de carbone voisins du noyau phényle,
R⁶ représente un alkyle en C₁ à C₄, halogène, ou trifluorométhyle,
R⁷ représente un alkyle en C₁ à C₄, halogène ou trifluorométhyle,
A représente un groupe -(CH₂)ₙ, dans lequel n représente un nombre entier compris entre 1 et 3, ou un groupe -NH-(CH₂)ₘ, où m représente un nombre entier de 2 ou 3, et
X est un groupe partant séparable.

10. Composés de formule générale X, 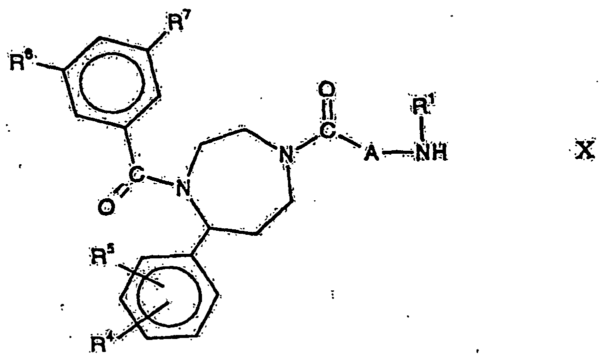 dans laquelle
R¹ représente un hydrogène ou un alkyle en C₁ à C₄,
R⁴ représente un hydrogène, alkyle en C₁ à C₄, alcoxy en C₁ à C₄, halogène ou trifluorométhyle, et
R⁵ représente un hydrogène, alkyle en C₁ à C₄, alcoxy en C₁ à C₄, halogène ou trifluorométhyle, ou
R⁴ et R⁵ représentent ensemble un alkylènedioxy ayant 1 ou 2 atomes de carbone lié aux atomes de carbone voisins du noyau phényle,
R⁶ représente un alkyle en C₁ à C₄, halogène, ou trifluorométhyle,
R⁷ représente un alkyle en C₁ à C₄, halogène ou trifluorométhyle, et
A représente un groupe -(CH₂)ₙ, dans lequel n représente un nombre entier de 1 à 3, ou un groupe -NH-(CH₂)ₘ, où m représente un nombre entier de 2 ou 3.
